(19) Europäisches Patentamt
European Patent Office
Office européen des brevets

(11) **EP 3 348 641 B1**

(12) **EUROPEAN PATENT SPECIFICATION**

(45) Date of publication and mention of the grant of the patent:
**18.12.2019 Bulletin 2019/51**

(21) Application number: **18159292.4**

(22) Date of filing: **10.09.2013**

(51) Int Cl.:
*C12N 15/09* (2006.01) *A61K 39/395* (2006.01)
*A61K 45/00* (2006.01) *A61P 35/00* (2006.01)
*C07K 14/705* (2006.01) *C12Q 1/68* (2018.01)
*G01N 33/15* (2006.01) *G01N 33/50* (2006.01)
*G01N 33/574* (2006.01) *A61K 31/713* (2006.01)
*C07K 16/28* (2006.01) *A61K 39/00* (2006.01)

(54) **CANCER MARKER AND APPLICATION THEREOF**

KREBSMARKER UND ANWENDUNG DAVON

MARQUEUR DE CANCER ET SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2012 JP 2012199508**

(43) Date of publication of application:
**18.07.2018 Bulletin 2018/29**

(62) Document number(s) of the earlier application(s) in accordance with Art. 76 EPC:
**13836385.8 / 2 896 692**

(73) Proprietor: **National University Corporation Kagawa University**
**Takamatsu-shi, Kagawa 760-8521 (JP)**

(72) Inventors:
- **NISHIYAMA, Akira**
  **Kagawa, 761-0793 (JP)**
- **SUZUKI, Fumiaki**
  **Gifu, 501-1193 (JP)**

(74) Representative: **J A Kemp LLP**
**14 South Square**
**Gray's Inn**
**London WC1R 5JJ (GB)**

(56) References cited:
**WO-A1-2012/045587** **WO-A1-2013/124406**
**WO-A2-99/34823** **US-A1- 2011 263 441**

- **L JUILLERAT-JEANNERET ET AL: "Renin and angiotensinogen expression and functions in growth and apoptosis of human glioblastoma", BRITISH JOURNAL OF CANCER, vol. 90, no. 5, 8 March 2004 (2004-03-08), pages 1059-1068, XP055313878, GB ISSN: 0007-0920, DOI: 10.1038/sj.bjc.6601646**

Remarks:
The file contains technical information submitted after the application was filed and not included in this specification

**EP 3 348 641 B1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

SEQUENCE LISTING

<110> NATIONAL UNIVERSITY CORPORATION KAGAWA UNIVERSITY

<120> Cancer marker and use thereof

<130> N405357EP-A

<150> JP2012-199508
<151> 2012-09-11

<160> 10

<170> PatentIn version 3.5

<210> 1
<211> 20
<212> DNA
<213> Artificial Sequence

<220>
<223> primer

<400> 1
ggcgttggtg gcgggtgttt 20

<210> 2
<211> 22
<212> DNA
<213> Artificial Sequence

<220>
<223> primer

<400> 2
agcccatgga caatgcagcc ac 22

<210> 3
<211> 24
<212> DNA
<213> Artificial Sequence

<220>
<223> primer

<400> 3
cacagagcct cgcctttgcc gatc 24

<210> 4
<211> 23

<212> DNA
<213> Artificial Sequence

<220>
<223> primer

<400> 4
acgagcgcgg cgatatcatc atc 23

<210> 5
<211> 2044
<212> DNA
<213> Homo sapiens

<400> 5

ctggacgagt ccgagcgcgt cacctcctca cgctgcggct gtcgcccgtg tcccgccggc 60
ccgttccgtg tcgccccgca gtgctgcggc cgccgcggca ccatggctgt gtttgtcgtg 120
ctcctggcgt tggtggcggg tgttttgggg aacgagttta gtatattaaa atcaccaggg 180
tctgttgttt tccgaaatgg aaattggcct ataccaggag agcggatccc agacgtggct 240
gcattgtcca tgggcttctc tgtgaaagaa gacctttctt ggccaggact cgcagtgggt 300
aacctgtttc atcgtcctcg ggctaccgtc atggtgatgg tgaagggagt gaacaaactg 360
gctctacccc caggcagtgt catttcgtac cctttggaga atgcagttcc ttttagtctt 420
gacagtgttg caaattccat tcactcctta ttttctgagg aaactcctgt tgttttgcag 480
ttggctccca gtgaggaaag agtgtatatg gtagggaagg caaactcagt gtttgaagac 540
ctttcagtca ccttgcgcca gctccgtaat cgcctgtttc aagaaaactc tgttctcagt 600
tcactccccc tcaattctct gagtaggaac aatgaagttg acctgctctt tctttctgaa 660
ctgcaagtgc tacatgatat ttcaagcttg ctgtctcgtc ataagcatct agccaaggat 720
cattctcctg atttatattc actggagctg gcaggtttgg atgaaattgg gaagcgttat 780
ggggaagact ctgaacaatt cagagatgct tctaagatcc ttgttgacgc tctgcaaaag 840
tttgcagatg acatgtacag tctttatggt gggaatgcag tggtagagtt agtcactgtc 900
aagtcatttg acacctccct cattaggaag acaaggacta tccttgaggc aaaacaagcg 960
aagaacccag caagtcccta taaccttgca tataagtata attttgaata ttccgtggtt 1020
ttcaacatgg tactttggat aatgatcgcc ttggccttgg ctgtgattat cacctcttac 1080
aatatttgga acatggatcc tggatatgat agcatcattt ataggatgac aaaccagaag 1140
attcgaatgg attgaatgtt acctgtgcca gaattagaaa aggggggttgg aaattggctg 1200
ttttgttaaa atatatcttt tagtgtgctt taaagtagat agtatacttt acatttataa 1260
aaaaaaatca aattttgttc tttattttgt gtgtgcctgt gatgtttttc tagagtgaat 1320
tatagtattg acgtgaatcc cactgtggta tagattccat aatatgcttg aatattatga 1380
tatagccatt taataacatt gatttcattc tgtttaatga atttggaaat atgcactgaa 1440
agaaatgtaa aacatttaga atagctcgtg ttatggaaaa aagtgcactg aatttattag 1500
acaaacttac gaatgcttaa cttctttaca cagcataggt gaaaatcata tttgggctat 1560
tgtatactat gaacaatttg taaatgtctt aatttgatgt aaataactct gaaacaagag 1620
aaaaggtttt taacttagag tagccctaaa atatggatgt gcttatataa tcgcttagtt 1680
ttggaactgt atctgagtaa cagaggacag ctgtttttta accctcttct gcaagtttgt 1740

tgacctacat gggctaatat ggatactaaa aatactacat tgatctaaga agaaactagc 1800

cttgtggagt atatagatgc ttttcattat acacacaaaa atccctgagg gacattttga 1860

ggcatgaata taaaacattt ttatttcagt aacttttccc cctgtgtaag ttactatggt 1920

ttgtggtaca acttcattct atagaatatt aagtggaagt gggtgaattc tactttttat 1980

gttggagtgg accaatgtct atcaagagtg acaaataaag ttaatgatga ttccaaaaaa 2040

aaaa 2044

<210> 6
<211> 350
<212> PRT
<213> Homo sapiens

<400> 6

```
Met Ala Val Phe Val Val Leu Leu Ala Leu Val Ala Gly Val Leu Gly
1               5                   10                  15

Asn Glu Phe Ser Ile Leu Lys Ser Pro Gly Ser Val Val Phe Arg Asn
            20                  25                  30

Gly Asn Trp Pro Ile Pro Gly Glu Arg Ile Pro Asp Val Ala Ala Leu
        35                  40                  45

Ser Met Gly Phe Ser Val Lys Glu Asp Leu Ser Trp Pro Gly Leu Ala
    50                  55                  60

Val Gly Asn Leu Phe His Arg Pro Arg Ala Thr Val Met Val Met Val
65                  70                  75                  80

Lys Gly Val Asn Lys Leu Ala Leu Pro Pro Gly Ser Val Ile Ser Tyr
                85                  90                  95

Pro Leu Glu Asn Ala Val Pro Phe Ser Leu Asp Ser Val Ala Asn Ser
            100                 105                 110

Ile His Ser Leu Phe Ser Glu Glu Thr Pro Val Val Leu Gln Leu Ala
        115                 120                 125

Pro Ser Glu Glu Arg Val Tyr Met Val Gly Lys Ala Asn Ser Val Phe
    130                 135                 140

Glu Asp Leu Ser Val Thr Leu Arg Gln Leu Arg Lys Arg Leu Phe Gln
145                 150                 155                 160

Glu Asn Ser Val Leu Ser Ser Leu Pro Leu Asn Ser His Ser Arg Asn
                165                 170                 175
```

```
Asn Glu Val Asp Leu Leu Phe Leu Ser Glu Leu Gln Val Leu His Asp
            180                 185                 190

Ile Ser Ser Leu Leu Ser Arg His Lys His Leu Ala Lys Asp His Ser
        195                 200                 205

Pro Asp Leu Tyr Ser Leu Glu Leu Ala Gly Leu Asp Glu Ile Gly Lys
    210                 215                 220

Arg Tyr Gly Glu Asp Ser Glu Gln Phe Arg Asp Ala Ser Lys Ile Leu
225                 230                 235                 240

Val Asp Ala Leu Gln Lys Phe Ala Asp Asp Met Tyr Ser Leu Tyr Gly
                245                 250                 255

Gly Asn Ala Val Val Glu Leu Val Thr Val Lys Ser Phe Asp Thr Ser
            260                 265                 270

Leu Ile Arg Lys Thr Arg Thr Ile Leu Glu Ala Lys Gln Ala Lys Asn
        275                 280                 285

Pro Ala Ser Pro Tyr Asn Leu Ala Tyr Lys Tyr Asn Phe Glu Tyr Ser
    290                 295                 300

Val Val Phe Asn Met Val Leu Trp Ile Met Ile Ala Leu Ala Leu Ala
305                 310                 315                 320

Val Ile Ile Thr Ser Tyr Asn Ile Trp Asn Met Asp Pro Gly Tyr Asp
                325                 330                 335

Ser Ile Ile Tyr Arg Met Thr Asn Gln Lys Ile Arg Met Asp
            340                 345                 350
```

<210> 7
<211> 14
<212> PRT
<213> Homo sapiens

<400> 7

```
His Lys His Leu Ala Lys Asp His Ser Pro Asp Leu Tyr Ser
1               5                   10
```

<210> 8
<211> 25
<212> RNA
<213> Artificial Sequence

<220>
<223> siRNA

<400> 8
uauagggacu ugcuggguuc uucgc 25

<210> 9
<211> 25
<212> RNA
<213> Artificial Sequence

<220>
<223> siRNA

<400> 9
gcgaagaacc cagcaagucc cuaua 25

<210> 10
<211> 350
<212> PRT
<213> Mus musculus

<400> 10

```
Met Ala Val Leu Val Val Leu Leu Phe Phe Leu Val Ala Gly Ala Leu
1               5                   10                  15

Gly Asn Glu Phe Ser Ile Leu Arg Ser Pro Gly Ser Val Val Phe Arg
            20                  25                  30

Asn Gly Asn Trp Pro Ile Pro Gly Asp Arg Ile Pro Asp Val Ala Ala
        35                  40                  45

Leu Ser Met Gly Phe Ser Val Lys Glu Asp Leu Ser Trp Pro Gly Leu
    50                  55                  60

Ala Val Gly Asn Leu Phe His Arg Pro Arg Ala Thr Ile Met Val Met
65                  70                  75                  80

Val Lys Gly Val Asp Lys Leu Ala Leu Pro Ala Gly Ser Val Ile Ser
                85                  90                  95

Tyr Pro Leu Glu Asn Ala Val Pro Phe Ser Leu Asp Ser Val Ala Asn
            100                 105                 110

Ser Ile His Ser Leu Phe Ser Glu Glu Thr Pro Val Val Leu Gln Leu
        115                 120                 125

Ala Pro Ser Glu Glu Arg Val Tyr Met Val Gly Lys Ala Asn Ser Val
    130                 135                 140

Phe Glu Asp Leu Ser Val Thr Leu Arg Gln Leu Arg Asn Arg Leu Phe
145                 150                 155                 160
```

```
Gln Glu Asn Ser Leu Leu Asn Ser Leu Pro Leu Asn Ser Leu Ser Arg
                165                 170                 175

Asn Asn Glu Val Asp Leu Leu Phe Leu Ser Glu Leu Gln Val Leu His
            180                 185                 190

Asp Ile Ser Ser Leu Leu Ser Arg His Lys His Leu Ala Lys Asp His
        195                 200                 205

Ser Pro Asp Leu Tyr Ser Leu Glu Leu Ala Gly Leu Asp Glu Leu Gly
    210                 215                 220

Lys Arg Tyr Gly Glu Asp Ser Glu Gln Phe Arg Asp Ala Ser Lys Ile
225                 230                 235                 240

Leu Val Asp Ala Leu Gln Lys Phe Ala Asp Asp Met Tyr Ser Leu Tyr
                245                 250                 255

Gly Gly Asn Ala Val Val Glu Leu Val Thr Val Lys Ser Phe Asp Thr
            260                 265                 270

Ser Leu Val Arg Lys Ser Arg Thr Ile Leu Glu Ala Lys Gln Glu Asn
        275                 280                 285

Thr Gln Ser Pro Tyr Asn Leu Ala Tyr Lys Tyr Asn Leu Glu Tyr Ser
    290                 295                 300

Val Val Phe Asn Leu Val Leu Trp Ile Met Ile Gly Leu Ala Leu Ala
305                 310                 315                 320

Val Ile Ile Thr Ser Tyr Asn Ile Trp Asn Met Asp Pro Gly Tyr Asp
                325                 330                 335

Ser Ile Ile Tyr Arg Met Thr Asn Gln Lys Ile Arg Ile Asp
            340                 345                 350
```

**Description**

Technical Field

The present invention relates to a cancer therapeutic drug.

Background Art

The commonest cause of death of Japanese has been cancer recently, and the leading cause of death in other countries also has been cancer. The emergence and the progress of cancer are related to various factors such as a genetic factor and an environmental factor. However, the definitive diagnosis and treatment methods have not been established yet.

Among cancers, for example, a pancreatic cancer is rapidly progressed, and thus, it is difficult to early detect the cancer, and at the time when the cancer is detected, it has already been metastasized to the surrounding lymph node, organ, and the like in many cases. As described above, the cancer has already been progressed at the stage of therapy. Thus, for example, there is a problem in that it is difficult to treat the cancer, and even when a surgery is performed, the prognosis is bad. Therefore, it is desired to develop a cancer marker which allows various cancers including the pancreatic cancer to be detected early (Non-patent documents 1 and 2).

Patent document 1 describes a method for diagnosing hypertension and cardiomyopathies.

Patent document 2 discloses compositions, kits, and methods for identification, assessment, prevention and therapy of hepatic disorders.

Patent document 3 discloses methods for preventing and treating fibrotic diseases resulting from accumulation of excess extracellular matrix induced by TGFβ using renin inhibitors.

Patent document 4 discloses a biomarker of neurodegeneration and neurogeneration.

Non-patent document 3 describes renin and angiotensinogen expression and functions in growth and apoptosis of human glioblastoma.

Prior Art Documents

Non-Patent Document

Non-Patent Document 1: Duffy MJ et al., "Tumor markers in pancreatic cancer: a European Group on Tumor Markers (EGTM) status report", Annals of Oncology, Oxford Journal, March 2010, Vol. 21, No. 3, p.441-447

Non-Patent Document 2: WATANABE Hiroyuki et al., "Feature, Diagnosis and treatment of pancreatic cancer, Is it possible to early diagnose pancreatic cancer? Tumor marker", Geka chiryo (surgical therapy), Nagai Shoten Co., Ltd., September 2007, Vol. 97, p.250-257

Non-Patent Document 3: Juillerat-Jeanneret, L et al. "Renin and angiotensinogen expression and functions in growth and apoptosis of human glioblastoma." British journal of cancer vol. 90,5 (2004): 1059-68.

Patent Documents

Patent Document 1: WO2012045587
Patent Document 2: US2011263441
Patent Document 3: WO9934823
Patent Document 4: WO2013124406

Summary of Invention

Problem to be Solved by the Invention

The present invention is intended to provide an antibody to a novel cancer marker or an antigen binding fragment thereof, and drugs for use in a method of treating cancer comprising administering the antibody or antigen-binding fragment.

Means for Solving Problem

The present invention provides an antibody to a prorenin receptor or an antigen binding fragment of the antibody, wherein

the antibody or the antigen binding fragment binds a region extending from the 200th to 213rd amino acid residues in an amino acid sequence (SEQ ID NO: 6) of a human prorenin receptor protein.

Also provided is a drug for use in a method of treating cancer, comprising the antibody or the antigen binding fragment according to the invention. The cancer may be a gastrointestinal cancer or a brain tumor. The gastrointestinal cancer may be at least one selected from the group consisting of a pancreatic cancer, a gastric cancer, a large bowel cancer and a liver cancer. The brain tumor may be at least one selected from the group consisting of glioma, astrocytoma, primary central nervous system malignant lymphoma and cavernous hemangioma.

Effects of the Invention

As the results of the earnest studies, the inventors of the present invention found that a prorenin receptor expression in a biological body showed a correlation with the occurrence of a cancer and thus arrived at the present invention. The finding allows a morbidity risk of a cancer in a subject to be tested by measuring a prorenin receptor expression level. Moreover, the prorenin receptor becomes a target of a cancer. Thus, by screening using the target, a candidate substance for use in a cancer therapy can also be obtained. Therefore, the antibody and antibody fragment of the present invention are really useful in a clinical field and a biochemical field.

Brief Description of Drawings

[FIG. 1] FIGs. 1A and 1B are graphs each showing the concentration of the prorenin receptor protein in the plasma in Example 1.
[FIG. 2] FIG. 2 is a graph showing the time-dependent change of the number of cells in Example 2.
[FIG. 3] FIG. 3 is a graph showing the expression of the prorenin receptor protein in Example 3.
[FIG. 4] FIG. 4 is a graph showing the expression of mRNA of the prorenin receptor gene in Example 3.
[FIG. 5] FIG. 5 is a graph showing the expression of the prorenin receptor protein in Example 4.
[FIG. 6] FIG. 6 is a graph showing the expression of mRNA of the prorenin receptor gene in Example 4.
[FIG. 7] FIG. 7 is a graph showing the expression of the prorenin receptor protein in Example 5.
[FIG. 8] FIG. 8 is a graph showing the expression of mRNA of the prorenin receptor gene in Example 5.
[FIG. 9] FIGs. 9A to 9D are tissue staining views each showing the expression of the prorenin receptor protein in Example 6.
[FIG. 10] FIGs. 10A and 10B are graphs each showing the tumor volume in Example 7.
[FIG. 11] FIGs. 11A and 11B are photographs of the mice in Example 7.
[FIG. 12] FIG. 12 is a graph showing the expression of the prorenin receptor protein in Example 7.
[FIG. 13] FIGs. 13A and 13B are graphs each showing the tumor volume in Example 8.
[FIG. 14] FIGs. 14A and 14B are photographs each showing the expression of the prorenin receptor protein in Example 9.
[FIG. 15] FIG. 15 is a photograph showing the expression of the prorenin receptor protein in Example 10.
[FIG. 16] FIG. 16 is a graph showing the expression of the phosphorylated LRP protein in Example 10.
[FIG. 17] FIGs. 17A to 17C are graphs each showing the expression of the active β-catenin protein and the Cyllin D1 protein in Example 10.
[FIG. 18] FIGs. 18A to 18C are graphs each showing the proliferation potency of cells in Example 11.
[FIG. 19] FIG. 19 shows dot plots each showing the proportion of the apoptosis cells in Example 12.
[FIG. 20] FIG. 20 is a histogram showing the proportion of the apoptosis cells in Example 12.
[FIG. 21] FIGs. 21A and 21B are graphs each showing the proportion of the cells in each cell cycle in Example 12.
[FIG. 22] FIGs. 22A to 22C are graphs each showing the activity of the caspase 3 in Example 12.
[FIG. 23] FIG. 23 is a graph showing the concentration of the prorenin receptor protein in plasma in Example 13.

Description of Embodiments

(Cancer marker)

The cancer marker according to the present disclosure is, as mentioned above, a prorenin receptor. The cancer marker according to the present disclosure allows a morbidity risk of a cancer in a subject to be tested by measuring a prorenin receptor expression level in a biological specimen obtained from the subject, for example.

The origin of the prorenin receptor is not limited to particular origins and can be set appropriately according to the type of a subject. Examples of the origin include humans and nonhuman animals except humans. Examples of the nonhuman animals include mammals such as a mouse, a rat, a dog, a monkey, a rabbit, a sheep, and a horse. For prorenin receptors derived from various animals, information registered in an existing database can be referred to. Specific examples of the prorenin receptors derived from humans include, as cDNA, a region (including a stop codon) extending from 103rd to 1155th bases in the following base sequence (SEQ ID NO: 5) registered as the NCBI accession No. NM_005765, and as a protein, the following amino acid sequence (SEQ ID NO: 6) registered as the NCBI accession No. NP_005756. The base sequence of SEQ ID NO: 5 is a sequence which encodes the amino acid sequence of SEQ ID NO: 6.

Human prorenin receptor cDNA (SEQ ID NO: 5)

ctggacgagtccgagcgcgtcacctcctcacgctgcggctgtcgcccgtgtcccgccggcccgttccgtgtcgccc
cgcagtgctgcggccgccgcggcaccatggctgtgtttgtcgtgctcctggcgttggtggcgggtgttttgggga
acgagtttagtatattaaaatcaccagggtctgttgttttccgaaatggaaattggcctataccaggagagcgg
atcccagacgtggctgcattgtccatgggcttctctgtgaaagaagacctttcttggccaggactcgcagtgggt
aacctgtttcatcgtcctcgggctaccgtcatggtgatggtgaagggagtgaacaaactggctctacccccagg
cagtgtcatttcgtaccctttggagaatgcagttccttttagtcttgacagtgttgcaaattccattcactccttatt
ttctgaggaaactcctgttgttttgcagttggctcccagtgaggaaagagtgtatatggtagggaaggcaaact
cagtgtttgaagacctttcagtcaccttgcgccagctccgtaatcgcctgtttcaagaaaactctgttctcagttc
actccccctcaattctctgagtaggaacaatgaagttgacctgctctttctttctgaactgcaagtgctacatgat
atttcaagcttgctgtctcgtcataagcatctagccaaggatcattctcctgatttatattcactggagctggcag
gtttggatgaaattgggaagcgttatggggaagactctgaacaattcagagatgcttctaagatccttgttgac
gctctgcaaaagtttgcagatgacatgtacagtctttatggtgggaatgcagtggtagagttagtcactgtcaa
gtcatttgacacctccctcattaggaagacaaggactatccttgaggcaaaacaagcgaagaacccagcaagt
ccctataaccttgcatataagtataattttgaatattccgtggttttcaacatggtactttggataatgatcgcctt
ggccttggctgtgattatcacctcttacaatatttggaacatggatcctggatatgatagcatcatttataggat
gacaaaccagaagattcgaatggattgaatgttacctgtgccagaattagaaaaggggggttggaaattggct
gttttgttaaaatatatcttttagtgtgctttaaagtagatagtatactttacatttataaaaaaaaaatcaaatt
ttgttctttattttgtgtgtgcctgtgatgtttttctagagtgaattatagtattgacgtgaatcccactgtggtata
gattccataatatgcttgaatattatgatatagccatttaataacattgatttcattctgtttaatgaatttggaa
atatgcactgaaagaaatgtaaaacatttagaatagctcgtgttatggaaaaaagtgcactgaatttattag
acaaacttacgaatgcttaacttctttacacagcataggtgaaaatcatatttgggctattgtatactatgaac
aatttgtaaatgtcttaatttgatgtaaataactctgaaacaagagaaaaggtttttaacttagagtagcccta
aaatatggatgtgcttatataatcgcttagttttggaactgtatctgagtaacagaggacagctgtttttttaacc
ctcttctgcaagtttgttgacctacatgggctaatatggatactaaaaatactacattgatctaagaagaaact
agccttgtggagtatatagatgcttttcattatacacacaaaaatccctgagggacattttgaggcatgaatat
aaaacatttttatttcagtaacttttccccctgtgtaagttactatggtttgtggtacaacttcattctatagaata
ttaagtggaagtgggtgaattctactttttatgttggagtggaccaatgtctatcaagagtgacaaataaagtt
aatgatgattccaaaaaaaaaa

Human prorenin receptor protein (SEQ ID NO: 6)

MAVFVVLLALVAGVLGNEFSILKSPGSVVFRNGNWPIPGERIPDVAALSM
GFSVKEDLSWPGLAVGNLFHRPRATVMVMVKGVNKLALPPGSVISYPLE
NAVPFSLDSVANSIHSLFSEETPVVLQLAPSEERVYMVGKANSVFEDLSV
TLRQLRKRLFQENSVLSSLPLNSHSRNNEVDLLFLSELQVLHDISSLLSRH
KHLAKDHSPDLYSLELAGLDEIGKRYGEDSEQFRDASKILVDALQKFADD
MYSLYGGNAVVELVTVKSFDTSLIRKTRTILEAKQAKNPASPYNLAYKYN
FEYSVVFNMVLWIMIALALAVIITSYNIWNMDPGYDSIIYRMTNQKIRMD

The prorenin receptor can be used as a cancer marker and can be used specifically as a cancer marker for a gastrointestinal cancer and a brain tumor, for example. Examples of the gastrointestinal cancer include a gastrointestinal tract cancer and a digestive gland cancer. Examples of the gastrointestinal tract cancer include a gastric cancer and a large bowel cancer, and examples of the digestive gland cancer include a pancreatic cancer and a liver cancer. Examples of the brain tumor include glioma, astrocytoma, primary central nervous system malignant lymphoma, and cavernous hemangioma. Besides these, the cancer marker according to the present disclosure can be a cancer marker for a peritoneal cancer, for example. Moreover, the present disclosure allows both of a primary cancer and a metastatic cancer to be tested, for example.

The marker according to the present disclosure may be, for example, a prorenin receptor protein or mRNA transcribed from a prorenin receptor gene.

(Test method for testing morbidity risk of cancer)

The test method for testing a morbidity risk of a cancer according to the present disclosure includes the step of measuring a prorenin receptor expression level in a biological specimen obtained from a subject, as mentioned above.

The present disclosure is characterized in that a prorenin receptor expression level is measured as a cancer marker, and the other steps and conditions are not limited to particular steps and conditions.

The test method according to the present disclosure allows a possibility of the occurrence of a cancer, the presence or absence of the occurrence of a cancer (whether or not malignant transformation occurs), the stage of a cancer, the state of prognosis, and the like to be evaluated, for example. Examples of a cancer to be tested include a gastrointestinal cancer and a brain tumor as mentioned above. Examples of the gastrointestinal cancer include a gastrointestinal tract cancer and a digestive gland cancer, examples of the gastrointestinal tract cancer include a gastric cancer and a large bowel cancer, and examples of the digestive gland cancer include a pancreatic cancer and a liver cancer. Examples of the brain tumor include glioma, astrocytoma, primary central nervous system malignant lymphoma, and cavernous hemangioma. The test method according to the present disclosure also allows a peritoneal cancer to be evaluated, for example. The present disclosure also allows both of a primary cancer and a metastatic cancer to be tested, for example.

In the test method according to the present disclosure, examples of the subject include humans and nonhuman animals except humans. Examples of the nonhuman animals include mammals such as a mouse, a rat, a dog, a monkey, a rabbit, a sheep, and a horse.

In the test method according to the present disclosure, the type of the biological specimen is not limited to particular specimens, and examples thereof include a body fluid, a cell derived from a body fluid, an organ, a tissue, and a cell, separated from a biological body. The body fluid can be, for example, blood, and specific examples thereof include whole blood, serum, and plasma. The cell derived from a body fluid can be, for example, a cell derived from blood, and specific examples thereof include blood cells such as a hemocyte, a leukocyte, and a lymphocyte. The biological specimen can be decided appropriately according to the type of a cancer to be tested, for example. The biological specimen is derived from an organ in which a cancer to be tested emerges, for example. Examples of the organ include a stomach, a pancreas, a large bowel, and a liver, a brain, and a peritoneum. Examples of the brain include a cerebrum, a temporal lobe, an occipital lobe, a cerebellum, a basal ganglion, and a mesenchymal tissue. As a specific example, in the case where the cancer to be tested is a pancreatic cancer, the biological specimen is preferably a tissue or a cell derived from a pancreas. In the case where the cancer to be tested is a brain tumor, the biological specimen is preferably a tissue or a cell derived from a brain. The cancer marker according to the present disclosure allows a cancer in a digestive organ such as a pancreas and a brain such as a cerebrum to be tested by a prorenin receptor expression level in blood, for example. Thus, the biological specimen is preferably whole blood, serum, or plasma, more preferably serum or plasma because burdens on subjects and doctors can be eased, for example.

A prorenin receptor expression to be measured can be, for example, an expression of mRNA of a prorenin receptor gene or a prorenin receptor protein. Either one of the expressions of the mRNA and the protein in the biological specimen may be measured, or both of them may be measured, for example. The methods for measuring these are not limited to particular methods, and known methods can be employed. Specific examples of the method for measuring the expression of the mRNA include gene amplification methods utilizing a reverse transcription reaction such as a reverse transcription (RT)-PCR method and the like. Specifically, for example, the method is a method in which cDNA is synthesized from mRNA by a reverse transcription reaction, and a gene amplification is performed using the cDNA as a template. Examples of the method for measuring the expression of the protein include an immune antibody method, an ELISA method, flow cytometry, and Western blotting.

The test method according to the present disclosure further includes the step of comparing a prorenin receptor expression level in the biological specimen (hereinafter also referred to the "biological specimen to be tested") obtained from the subject with a reference value to test the morbidity risk of the cancer in the subject. The reference value is not limited to particular values, and examples thereof include the prorenin receptor expression levels in a healthy subject, a cancer patient, and a cancer patient in each stage of progress. In the case of evaluating the prognosis, the reference value may be the prorenin receptor expression level after therapy (for example, immediately after therapy) in the same subject, for example.

The reference value can be obtained using a biological specimen (hereinafter also referred to as a "reference biological specimen") isolated from a healthy subject and/or a cancer patient as mentioned above, for example. In the case of evaluating the prognosis, a reference biological specimen isolated from the same subject after therapy may be used, for example. The reference value may be measured at the same time as the measurement of the biological specimen to be tested obtained from the subject or in advance. The latter case is preferable because it is not necessary to obtain the reference value every time the measurement of the biological specimen to be tested obtained from the subject. It is preferred that the biological specimen to be tested obtained from the subject and the reference biological specimen are collected under the same conditions and subjected to the measurement of a prorenin receptor under the same conditions, for example.

In the step of comparing, a method for evaluating a morbidity risk of a cancer in a subject is not limited to particular methods and can be decided appropriately according to the type of the reference value. As a specific example, when the prorenin receptor expression level in a biological specimen to be tested obtained from a subject is significantly higher than that in a reference biological specimen obtained from a healthy subject, identical to (has no significant difference from) that in a reference biological specimen obtained from a cancer patient, and/or is significantly higher than that in a reference biological specimen obtained from a cancer patient, it can be evaluated that the subject has a morbidity risk or a high morbidity risk of a cancer. When the prorenin receptor expression level in a biological specimen to be tested obtained from a subject is identical to (has no significant difference from) that in a reference biological specimen obtained from a healthy subject, is significantly lower than that in a reference biological specimen obtained from a healthy subject, and/or is significantly lower than that in a reference biological specimen obtained from a cancer patient, it can be evaluated that the subject has no morbidity risk or a low morbidity risk of a cancer. In the step of comparing, comparing the prorenin receptor expression level in a biological specimen to be tested obtained from a subject with that in a reference biological specimen obtained from a cancer patient in each stage of progress allows the degree of progress of a cancer to be evaluated. Specifically, when the expression level in a biological specimen to be tested obtained from a subject is, for example, almost identical to (has no significant difference from) that in the reference biological specimen in any stage of progress, it can be evaluated that the subject has a possibility of being in the stage of progress.

In the case where the state of prognosis is evaluated in the step of comparing, the evaluation may be performed in the same manner as mentioned above or can be performed using, as a reference value, the prorenin receptor expression level in a reference biological specimen obtained from the same subject after therapy, for example. As a specific example, when the prorenin receptor expression level in a biological specimen to be tested obtained from a subject is significantly higher than the reference value, it can be evaluated that the subject has a risk of relapse or deterioration after therapy. When the prorenin receptor expression level in a biological specimen to be tested obtained from a subject is identical to (has no significant difference from) the reference value and/or is significantly lower than the reference value, it can be evaluated that the subject has no risk of a low risk of relapse after therapy.

According to the present disclosure, the prorenin receptor expression levels in biological specimens collected over time from the same subject may be compared, for example. Then, for example, when the expression level is increased over time, it can be determined that the morbidity risk is increased and the like, and when the expression level is decreased over time, it can be determined that the morbidity risk is decreased, the cancer is cured, and the like.

(Test reagent)

The test reagent according to the present disclosure is a test reagent for use in the test method for testing a morbidity risk of a cancer according to the present disclosure, including an expression measurement reagent for measuring a prorenin receptor expression. The test reagent according to the present disclosure allows the test method for testing a morbidity risk of a cancer according to the present disclosure to be performed easily. The present disclosure is characterized in that a test of a morbidity risk of a cancer is performed on the basis of the measurement of a prorenin receptor expression and is only required that the prorenin receptor expression is measured, and the type of the expression measurement reagent is not limited to particular reagents. The expression measurement reagent for measuring a prorenin receptor expression may be, for example, a reagent for measuring an expression of a prorenin receptor protein or mRNA of a prorenin receptor gene.

The former can be, for example, a substance for binding to a prorenin receptor protein, and a specific example thereof can be an antibody. In this case, the test reagent according to the present disclosure preferably further includes a detection reagent for detecting a binding between the prorenin receptor protein and the antibody, for example. The detection reagent can be, for example, a combination of a detectable labeled antibody to the antibody and a substrate to the label.

The latter can be, for example, a reagent for amplifying mRNA of a prorenin receptor gene by a reverse transcription, and a specific example thereof can be, for example, a primer. The primer can be designed appropriately based on a gene sequence of a prorenin receptor, for example.

(Cancer diagnostic method and Cancer diagnostic reagent)

A cancer diagnostic method for diagnosing a cancer according to the present disclosure includes the step of measuring a prorenin receptor expression level in a biological specimen obtained from a subject. A cancer diagnostic reagent for diagnosing a cancer according to the present disclosure includes an expression measurement reagent for measuring a prorenin receptor expression. The present disclosure can be described with reference to the descriptions of the test method and the test reagent according to the present disclosure.

(Cancer therapeutic drug and use in a method of treating cancer)

A target of a cancer according to the present invention is a prorenin receptor. A prorenin receptor can be used as the target of a cancer, and for example, the cancer can be treated by suppressing an expression of mRNA of a prorenin receptor gene or a prorenin receptor protein or suppressing or neutralizing a function of a prorenin receptor protein, and the like.

The cancer therapeutic drug according to the present invention includes an antibody to a prorenin receptor or antigen binding fragment of the antibody. Also disclosed is an expression suppressive substance for suppressing a prorenin receptor expression as mentioned above. The cancer therapeutic is for use in a method for treating cancer.

The antibody or antigen binding fragment of the the present invention treats cancer by binding to a prorenin receptor protein

The antibody is not limited to particular antibodies and can be obtained by a general method for producing an antibody. As a specific example, an animal is immunized with a prorenin receptor protein or a fragment thereof as an antigen, and serum is collected from the immunized animal, and thus, an antibody to the prorenin receptor protein can be obtained. The antibody may be, for example, a monoclonal antibody or a polyclonal antibody.

The antigen may be a partial sequence of a prorenin receptor as mentioned above. The partial sequence is a region extending from the 200th to 213rd amino acid residues (HKHLAKDHSPDLYS:SEQ ID NO: 7) in an amino acid sequence (SEQ ID NO: 6) of a human prorenin receptor protein.

The antibody or antigen binding fragment of the present invention, binds to a human prorenin receptor protein, and the position of the binding is a region extending from 200th to 213rd amino acid residues (HKHLAKDHSPDLYS:SEQ ID NO: 7) in the amino acid sequence (SEQ ID NO: 6) of the human prorenin receptor protein.

Examples of expression suppressive substances include a substance for suppressing a transcription of mRNA from a prorenin receptor gene, a substance for cleaving transcribed mRNA, and a substance for suppressing a translation of a protein from mRNA. Specific examples thereof include an RNA interference reagent such as siRNA, an antisense, and a ribozyme, and they may be used alone or in a combination of two or more of them.

The conditions under which the cancer therapeutic drug according to the present invention is administrated are not limited to particular conditions, and the type of administration, the timing of administration, the amount of administration, and the like can be set appropriately according to the type of a target cancer disease, the degree of progress, the age of the subject, and the like. Examples of the target to be administrated include humans and nonhuman animals except humans. Examples of the nonhuman animals include mammals such as a mouse, a rat, a dog, a monkey, a rabbit, a sheep, and a horse.

(Screening method)

The screening method according to the present disclosure is a screening method for a cancer therapeutic drug candidate substance(s), including selecting, from a target substance(s), a binding substance for binding to a prorenin receptor or an expression suppressive substance for suppressing a prorenin receptor expression as the cancer therapeutic drug candidate substance(s). The present disclosure is characterized in that a target of the cancer therapeutic drug candidate substance is a prorenin receptor, and the other steps and conditions are not limited to particular steps and conditions.

Examples of the binding substance of the disclosure include a low molecular weight compound, a peptide, a protein, and a nucleic acid, and the binding substance is preferably an antibody or an antigen binding fragment.

Examples of the expression suppressive substance include a substance for suppressing a transcription of mRNA from a prorenin receptor gene, a substance for cleaving transcribed mRNA, and a substance for suppressing a translation of a protein from mRNA. Specific examples thereof include an RNA interference reagent such as siRNA, an antisense, and a ribozyme.

A screening method for the binding substance according to the present disclosure includes the steps of causing a target substance(s) to be in contact with a prorenin receptor; detecting a binding between the prorenin receptor and the target substance(s); and selecting the target substance(s) binding to a prorenin receptor as the cancer therapeutic drug candidate substance(s). In the step of detecting, a method for detecting a binding between the prorenin receptor and the target substance(s) is not limited to particular methods and can be decided appropriately according to the type of the target substance(s), for example.

A screening method for the expression suppressive substance according to the present disclosure includes the steps of: causing a target substance(s) to be present in an expression system of the prorenin receptor to cause a prorenin receptor expression; detecting the prorenin receptor expression in the expression system; and selecting the target substance(s) having a prorenin receptor expression level lower than an expression system of a control including no target substance present therein as the candidate substance(s), for example. In the step of detecting, the expression to be detected may be, for example, an expression of a prorenin receptor protein or a transcription of mRNA of a prorenin receptor gene. Methods for detecting the expression of the protein and the expression of the mRNA is not limited to particular methods, and known methods can be employed.

Examples

The examples of the present invention will be described below. It is to be noted, however, that the present invention is not limited by the following examples.

[Example 1]

The increase in the concentration of the prorenin receptor in the plasma of pancreatic cancer patients was examined.

The measurement of the prorenin receptor in the plasma was performed using Human soluble (Pro)renin Receptor Assay Kit (product of Immuno-Biological Laboratories Co. Ltd) according to its protocol. First, plasma was collected from the blood of each of the following subjects: healthy males (n = 9), healthy females (n = 3), male pancreatic cancer patients (n = 9), and female pancreatic cancer patients (n = 8). The collected plasma was diluted 2-fold with the diluent (phosphate buffer solution that contains 1% BSA and 5% Tween-20) of the kit, and the diluted plasma thus obtained was used as a test sample. 100 μl of the test sample was added to a plate to which a capture antibody (anti-Human renin receptor polyclonal rabbit IgG antibody) is bound, and allowed to react at 4°C overnight. The test sample was removed and the plate was washed four times with the phosphate buffer solution. Next, 100 μl of labeled antibody (HRP-labeled anti-Human renin receptor polyclonal mouse IgG antibody) was added to the plate and allowed to react at 4°C for 60 minutes. The labeled antibody was removed and the plate was washed five times with the phosphate buffer solution. Furthermore, after developing the color by adding 100 μl of a Tetra Methyl Benzidine-containing substrate solution to the plate, the color development reaction was stopped with 0.5 mol/l (1N) $H_2SO_4$. After stopping the reaction, the absorbance at 450 nm regarding the reacted solution in the plate was measured by a plate reader. On the other hand, the standard (Human soluble (Pro)renin Receptor) of the kit was serially diluted, and the absorbance was measured in the same manner using each of the thus obtained diluted standard samples to make a calibration curve. Then, from the absorbance of the test sample, the concentration of the prorenin receptor in the plasma was measured on the basis of the calibration curve.

Also plasma was collected from the following subjects: healthy subjects (n = 20), pancreatic cancer patients of primary pancreatic cancer without metastasis (no metastasis, n = 11), and pancreatic cancer patients of primary pancreatic cancer with metastases to other organs (with metastases, n = 9), and the concentration of the prorenin receptor in the plasma was measured in the same manner.

The results thereof are shown in FIGs. 1A and 1B. FIGs. 1A and 1B are graphs each showing the concentration of the prorenin receptor in the plasma. In FIGs. 1A and 1B, each of the horizontal axes indicates the types of the test samples and each of the vertical axes indicates the concentration of the prorenin receptor. As can be seen from FIG. 1A, in both male and female subjects, the concentrations of the prorenin receptor in the plasma of the pancreatic cancer patients (PDAC) were significantly higher than those in the plasma of the healthy subjects (Normal). As can be seen from FIG. 1B, in both the pancreatic cancer patients without metastasis and the pancreatic cancer patients with metastases, the concentrations of the prorenin receptor in the plasma were significantly higher than those in the healthy subjects. From these results, it was found that there is a correlation between the occurrence of the pancreatic cancer and the increase in the concentration of the prorenin receptor in the plasma regardless of the presence or absence of metastasis or sex, which demonstrates that the prorenin receptor in the plasma serves as a pancreatic cancer marker.

[Example 2]

The prorenin receptor expression in pancreatic cancer cell lines was suppressed and the proliferative potency of the cell line was examined.

Human pancreatic cancer cell line PK-1 with 40% confluence was cultured in a serum-free culture medium on a dish under the conditions of 37°C and 5% $CO_2$ for 24 hours. Next, PK-1 was transfected with the following siRNA (P) RR (product of Life Technologies) for causing knockdown of a prorenin receptor gene or scrambled siRNA (product of Life Technologies), which is a negative control, using Lipofectamine (trademark) RNAiMAX (product of Life Technologies) according to its protocol. Then, siRNA-transfected PK-1 was cultured under the same conditions for 48 hours. After the culture, PK-1 was peeled from the dish using 0.25% trypsin and 1 mmol/l EDTA-containing phosphate buffer solution (product of Life Technologies), and the serum-free culture medium was newly added. Then, from the culture medium that contains PK-1, a pellet that contains PK-1 was collected by centrifugation. The pellet was suspended in 1 ml of phosphate buffer solution, the suspension thus obtained and 20 μl of trypan blue solution were mixed, and the resultant was injected into a hemocytometer to calculate the number of cells per 1 ml. The resultant was seeded so as to achieve the number of living cells of $5 \times 10^5$ cells/10 cm-diameter dish, and human Wnt3a (product of R&D Systems) for inducing cell proliferation was added at 150 ng/ml. After the culture of 0, 24, 48, and 72 hours, PK-1 was collected and the number of living cells was calculated in the same manner. Also, as a control, the culture and the measurement of the number of living cells were performed in the same manner except that the cell line was not treated with siRNA.

siRNA (P) RR

Sense strand (SEQ ID NO: 8)
5'-UAUAGGGACUUGCUGGGUUCUUCGC-3'
Antisense strand (SEQ ID NO: 9)
5'-GCGAAGAACCCAGCAAGUCCCUAUA-3'

The results thereof are shown in FIG. 2. FIG. 2 is a graph showing the number of cells of the pancreatic cancer cell line when the prorenin receptor expression is suppressed by RNAi. In FIG. 2, the control (filled circle) indicates the results of a control group not treated with siRNA, the scramble (filled triangle) indicates the results of a scramble group treated with scramble siRNA, and siRNA (P) RR (filled square) indicates the results of a siRNA (P) RR group treated with siRNA (P) RR. In FIG. 2, the horizontal axis indicates the time stimulated by Wnt3a and the vertical axis indicates the number of cells. As can be seen from FIG. 2, the control group and the scramble group showed comparable cell proliferation at each processing time. In contrast, the cell proliferation of the pancreatic cancer cell line of the siRNA (P) RR group was suppressed as compared with that of the control group and the scramble group at every processing time. Furthermore, the cells in the control group and the scramble group were continuously proliferated up to 72 hours whereas the number of cells in the siRNA (P) RR group was decreased after peaking at 48 hours. From this result, it was found that the proliferation of the pancreatic cancer cell line can be suppressed by suppressing the prorenin receptor expression. That is, this result showed that the prorenin receptor can be a pancreatic cancer target.

[Example 3]

The increase in the expression of the prorenin receptor protein and mRNA in metastatic cancer tissues was examined.

(1) Expression of prorenin receptor protein

From cancer patients of primary pancreas cancer, normal liver tissues (control), metastatic liver cancer tissues, and metastatic peritoneal cancer tissues were collected. 1 ml of Lysis buffer was added to 2 g of each tissue, and the resultant was subjected to homogenization at 4,000 rpm for 5 minutes to prepare a test sample. The amount of the protein in the test sample was measured using a reagent (trade name: Bio-Rad Protein Assay (product of Bio-Rad)).

The test sample (total protein amount: 20 μg) was subjected to electrophoresis using 10% SDS polyacrylamide gel and transcribed to a polyvinylidene difluoride (PVDF) membrane. The membrane after the transcription was subjected to blocking using Blocking buffer (product of Li-Cor BioSciences). The membrane after the blocking was allowed to react with 1000-fold diluted anti-rabbit (P) RR polyclonal antibody at room temperature for 1 hour, followed by reaction with 2000-fold diluted IRDye (registered trademark)-labeled anti-rabbit IgG antibody (product of Li-Cor BioSciences) under the same conditions. Regarding the membrane after reaction, the expression level of the prorenin receptor protein in the test sample was measured using Odyssey (registered trademark) System (product of Li-Cor BioSciences). The relative value of the expression levels of the metastatic liver cancer tissue and the metastatic peritoneal cancer tissue was obtained assuming that the expression level of the normal liver tissue, which is a control, was 1.

The results of the expression of the prorenin receptor protein in the respective tissues are shown in FIG. 3. FIG. 3 is a graph showing the expression levels of the prorenin receptor proteins in the respective tissues. The horizontal axis indicates the types of test samples and the vertical axis indicates the relative expression level of the prorenin receptor protein. As can be seen from FIG. 3, the expression of the prorenin receptor protein in the metastatic liver cancer tissues and the metastatic peritoneal cancer tissues was significantly higher than that in the normal liver tissues. From these results, it was found that there is a correlation between the metastatic cancer tissue and the increase in the expression of the prorenin receptor protein, which demonstrates that the prorenin receptor protein serves as a metastatic cancer marker.

(2) Expression of mRNA of prorenin receptor gene

The normal liver tissues (control), the metastatic liver cancer tissues, and the metastatic peritoneal cancer tissues collected in the above item (1) were used. 1 ml of ISOGEN (product of Nippon Gene) was added to 2 g of each tissue, and subjected to homogenization at 4,000 rpm for 5 minutes to prepare a test sample. Ethanol was added to the test sample so as to precipitate RNA, and the RNA was dissolved in RNase free water to prepare a RNA solution. The concentration and $OD_{260}/OD_{280}$ (purity) of the RNA solution were measured by a spectrophotometer. Then, the RNA solution having a RNA concentration of 300 ng/μl with a purity of 1.8 or more was used for the quantitative real time (qRT)-PCR below.

After synthesizing cDNA from the RNA solution by a routine procedure using reverse transcriptase and random primers, RNA was degraded using RNaseH. With the cDNA thus obtained being used as a template, qRT-PCR was performed using SYBR (registered trademark) Green (product of Applied Biosystems) and Applied Biosystems 7300 Real-Time PCR System (product of Applied Biosystems) according to the attached protocols, and the expression level of mRNA of the prorenin receptor gene in the test sample and the expression level of mRNA of the β-actin gene, which is an internal standard, were measured. The expression level of mRNA of the prorenin receptor gene was calculated as a ratio to the expression level of mRNA of the β-actin gene. In the qRT-PCR, the respective primer sets below were used for amplifying the prorenin receptor gene and the β-actin gene.

Prorenin receptor gene amplification primer set

(SEQ ID NO: 1) 5'-GGCGTTGGTGGCGGGTGTTT-3'
(SEQ ID NO: 2) 5'-AGCCCATGGACAATGCAGCCAC-3'

β-actin gene amplification primer set

(SEQ ID NO: 3) 5'-CACAGAGCCTCGCCTTTGCCGATC-3'
(SEQ ID NO: 4) 5'-ACGAGCGCGGCGATATCATCATC-3'

The results of the expression levels of mRNA of the prorenin receptor gene in the respective tissues are shown in FIG. 4. The horizontal axis indicates the types of test samples and the vertical axis indicates the relative expression level of mRNA of the prorenin receptor gene to mRNA of the β-actin gene. As can be seen from FIG. 4, the expression of mRNA of the prorenin receptor gene in the metastatic liver cancer tissues and the metastatic peritoneal cancer tissues was higher than that in the normal liver tissues. From this result, it was found that there is a correlation between the metastatic cancer tissue and the increase in the expression of mRNA of the prorenin receptor gene, which demonstrates that mRNA of the prorenin receptor gene serves as a metastatic cancer marker.

[Example 4]

The increase in the expression of the prorenin receptor protein and mRNA in gastric cancer tissues was examined.

(1) Expression of prorenin receptor protein

Normal gastric tissues (n = 4) and gastric cancer tissues (n = 4) were collected from four gastric cancer patients, and the expression level of the prorenin receptor protein was measured in the same manner as in the item (1) in Example 3. Then, the average for the expression levels was obtained regarding each of the normal tissues and the cancer tissues of four subjects.

The results of the expression of the prorenin receptor protein in the gastric cancer tissue are shown in FIG. 5. FIG. 5 is a graph showing the expression level of the prorenin receptor protein. The horizontal axis indicates the types of test samples and the vertical axis indicates the relative expression level of the prorenin receptor protein. As can be seen from FIG. 5, the expression of the prorenin receptor protein in the gastric cancer tissues (n = 4) was significantly higher than that in the normal gastric tissues (n = 4). From these results, it was found that there is a correlation between the occurrence of the gastric cancer and the increase in the expression of the prorenin receptor protein, which demonstrates that the prorenin receptor protein in the gastric tissue serves as a gastric cancer marker.

(2) Expression of mRNA of prorenin receptor gene

The normal gastric tissues and the gastric cancer tissues collected in the above item (1) were used, and the expression level of mRNA of the prorenin receptor gene was measured in the same manner as in Example 3(2). Then, the average for the expression levels was obtained regarding each of the normal tissues and the cancer tissues of four subjects.

The results of the expression level of mRNA of the prorenin receptor gene in the gastric cancer tissue are shown in FIG. 6. The horizontal axis indicates the types of test samples and the vertical axis indicates the relative expression level of mRNA of the prorenin receptor gene to mRNA of the β-actin gene. As can be seen from FIG. 6, the expression of mRNA of the prorenin receptor gene in the gastric cancer tissues (n = 4) was higher than that in the normal gastric tissues (n = 4). From this result, it was found that there is a correlation between the occurrence of the gastric cancer and the increase in the expression of mRNA of the prorenin receptor gene, which demonstrates that the mRNA of the prorenin receptor gene in the gastric tissue serves as a gastric cancer marker.

[Example 5]

The increase in the expression of the prorenin receptor protein and mRNA in large bowel cancer tissues was examined.

(1) Expression of prorenin receptor protein

Normal large bowel tissues (n = 4) and large bowel cancer tissues (n = 4) were collected from four large bowel cancer patients, and the expression level of the prorenin receptor protein was measured in the same manner as in the item (1) in Example 3. Then, the average for the expression levels was obtained regarding each of the normal tissues and the cancer tissues of four subjects.

The results of the expression of the prorenin receptor protein in the large bowel cancer tissue are shown in FIG. 7. FIG. 7 is a graph showing the expression level of the prorenin receptor protein. The horizontal axis indicates the types of test samples and the vertical axis indicates the relative expression level of the prorenin receptor protein. As can be seen from FIG. 7, the expression of the prorenin receptor protein in the large bowel cancer tissues (n = 4) was significantly higher than that in the normal large bowel tissues (n = 4). From these results, it was found that there is a correlation between the occurrence of the large bowel cancer and the increase in the expression of the prorenin receptor protein, which demonstrates that the prorenin receptor protein in the large bowel tissue serves as a large bowel cancer marker.

(2) Expression of mRNA of prorenin receptor gene

Regarding the normal large bowel tissues and the large bowel cancer tissues collected in the above item (1), the expression level of mRNA of the prorenin receptor gene was measured in the same manner as in Example 3(2). Then, the average for the expression levels was obtained respectively regarding the normal tissues and the cancer tissues of four subjects.

The results of the expression level of mRNA of the prorenin receptor gene in the large bowel cancer tissue are shown in FIG. 8. The horizontal axis indicates the types of test samples and the vertical axis indicates the relative expression level of mRNA of the prorenin receptor gene to mRNA of the β-actin gene. As can be seen from FIG. 8, the expression of mRNA of the prorenin receptor gene in the large bowel cancer tissues (n = 4) was significantly higher than that in the normal large bowel tissues (n = 4). From this result, it was found that there is a correlation between the occurrence of the large bowel cancer and the increase in the expression of mRNA of the prorenin receptor gene, which demonstrates that mRNA of the prorenin receptor gene in the large bowel tissue serves as a large bowel cancer marker.

[Example 6]

The increase in the expression of the prorenin receptor protein in pancreatic cancer tissues was examined by immunohistochemical staining.

From twenty-two pancreatic cancer patients, normal epithelial tissues of the pancreatic duct, pancreatic cancer tissues, and epithelial tissues of the pancreatic duct containing preneoplastic lesion (PanIN) were collected. Paraffin sections were prepared with these tissues as test samples, and deparaffinization was performed using 100% xylene and 99.5% ethanol. Then, regarding each sample, endogenous peroxidase was inactivated using 100% ethanol and 30% hydrogen peroxide water. Next, after enclosing the section area of the test sample with a liquid blocker (water-repellent material), the sample was washed with phosphate buffer saline (PBS). Subsequently, the test sample was subjected to blocking at room temperature for 15 minutes using 10% normal goat serum (trade name: Histofine SAB-PO (R) kit, product of: NICHIREI BIOSCIENCES INC.).

After the blocking, the test sample was allowed to react at room temperature for 1 hour with 4000-fold diluted anti-rabbit (P) RR polyclonal antibody and then washed with PBS. Next, the test sample was allowed to react at room temperature for 30 minutes with Histofine Simple Stain MAX-PRO (MULTI) (product of: NICHIREI BIOSCIENCES INC.), which is a HRP-labeled secondary antibody. Subsequently, the test sample was washed with PBS and the color was developed with 3,3'-diaminobenzidine (DAB), which is a HRP coloring substrate. Then, the test sample was washed with running water and stained by haematoxylin and eosin (H&E) staining. The test sample after staining was washed with running water, dewatered with 99.5% ethanol and 100% xylene, and then immobilized. The test sample immobilized was sealed with marinol, and the expression site of the prorenin receptor stained brown with DAB was observed using an optical microscope.

The results of the expression of the prorenin receptor protein in the pancreatic tissue are shown in FIGs. 9A to 9D. FIGs. 9A to 9D are tissue staining views each showing the expression of the prorenin receptor protein in the pancreatic tissue. FIG. 9A is a tissue staining view of the pancreatic cancer tissue, FIG. 9B is a tissue staining view of the normal epithelial tissue of the pancreatic duct, FIG. 9C is a tissue staining view of the epithelial tissue of the pancreatic duct containing PanIN-2, which is the middle stage of PanIN, and FIG. 9D is a tissue staining view of the epithelial tissue of the pancreatic duct containing PanIN-3, which is the late stage of PanIN. In FIGs. 9A to 9D, each of the scale bars has a scale unit of 100 μm, and the stained areas are enclosed by solid lines (for example, the areas indicated by arrows X, Y, and Z).

As can be seen from FIG. 9B, brown staining was hardly observed regarding the epithelial tissue of the pancreatic duct (normal). On the other hand, as can be seen from FIG. 9A, the area enclosed by the solid line (X) was stained brown in the pancreatic cancer tissue. Furthermore, as can be seen from FIGs. 9C and 9D, the respective areas enclosed by the solid lines (Y) and (Z) in the PanIN-2 and PanIN-3 were stained brown. That is, the expression of the prorenin receptor protein in the pancreatic cancer tissue and PanIN was higher than that in the normal epithelial tissue of the pancreatic duct. It is to be noted that although FIGs. 9A to 9D show tissue staining views of one pancreatic cancer patient, the expression of the prorenin receptor protein in the pancreatic cancer tissue and PanIN was increased in the same manner also in the tissues of other pancreatic cancer patients. From these results, it was found that there is a correlation of the formation of PanIN and the occurrence of the pancreatic cancer with the increase in the expression of the prorenin receptor protein, which demonstrates that the prorenin receptor protein in the pancreatic tissue serves as a marker showing high possibility of occurring the pancreatic cancer and a pancreatic cancer marker.

[Example 7]

Human pancreatic cancer cell lines treated with siRNA (P) RR were transplanted to nude mice, and the suppression of growth of the pancreatic cancer cell lines and the decrease in the expression level of soluble prorenin receptor protein in the plasma in the nude mice were examined.

(1) Growth of pancreatic cancer cell line

siRNA (P) RR was transfected in the same manner as in Example 2 except that PANC-1, which is a human pancreatic cancer cell line, was used instead of PK-1, and PANC-1 (siRNA (P) RR group) transfected with siRNA was collected. Next, PANC-1 having $5 \times 10^5$ cells was subcutaneously transplanted to the upper right flank of 5-week male BALB/c nude mouse (n=7) (product of CLEA Japan Inc.). Then, the major axis and minor axis of PANC-1-derived tumor in the upper right flank were measured using a micrometer (AS ONE corporation) 25 and 40 days after the transplantation. The tumor volume was calculated by the equation below. As a control, the tumor volume was measured in the same manner except that PANC-1 (scramble group) transfected with scrambled siRNA was used instead of siRNA (P) RR.

[Equation 1]

$$\text{Cancer volume (mm}^3\text{)} = \text{major axis} \times (\text{minor axis})^2 \times 0.5$$

FIGs. 10A and 10B show the tumor volume and FIGs. 11A and 11B show the photographs of the mice 25 days after the transplantation. FIG. 10A is a graph showing the tumor volume 25 days after the transplantation and FIG. 10B is a graph showing the tumor volume 40 days after the transplantation. In FIGs. 10A and 10B, each of the horizontal axes indicates the types of siRNA and each of the vertical axes indicates the tumor volume. As can be seen from FIGs. 10A and 10B, the siRNA(P) RR group-derived tumor volume 25 and 40 days after the transplantation was significantly lower than that of the scramble group-derived tumor.

FIG. 11A is a photograph of the mouse 25 days after the scramble group transplantation and FIG. 11B is a photograph of the mouse 25 days after the si (P) RR group transplantation. As can be seen from FIGs. 11A and 11B, the formation of a tumor was observed in the area enclosed by the solid line (T) regarding the mouse 25 days after the scramble group transplantation. In contrast, no formation of a tumor was observed regarding the siRNA (P) RR group. From these results, it was found that the growth of the tumor in a biological body can be suppressed by suppressing the expression of the prorenin receptor.

(2) Expression level of prorenin receptor protein

Plasma was collected from the blood of the nude mouse 25 days after the transplantation. The expression level of the prorenin receptor protein in the plasma was measured in the same manner as in the item (1) in Example 3. Next, assuming that the expression level of the prorenin receptor in the plasma of the control was 1, the relative value of the expression level of the prorenin receptor in the plasma of the nude mouse transplanted with the siRNA (P) RR-transfected PANC-1 was obtained.

The results thereof are shown in FIG. 12. FIG. 12 is a graph showing the relative value of the expression level of the prorenin receptor in the plasma 25 days after the transplantation. In FIG. 12, the horizontal axis indicates the types of siRNA and the vertical axis indicates the relative expression level of the prorenin receptor. As can be seen from FIG. 12, the relative expression level of the prorenin receptor in the plasma of the nude mouse transplanted with the siRNA (P) RR group was significantly lower than that of the nude mouse transplanted with the scramble group. From these results, it was found that the expression level of the prorenin receptor in the plasma can be suppressed by suppressing the expression of the prorenin receptor of the tumor in the biological body.

[Example 8]

Whether the growth of pancreatic cancer cells transplanted into nude mice can be suppressed by administrating an anti-prorenin receptor antibody to the mice was examined.

(1) Preparation of anti-prorenin receptor antibody

An antigen protein was prepared by binding a polypeptide (SEQ ID NO: 7) extending from the 200th to 213rd amino acid residues in the human prorenin receptor protein (SEQ ID NO: 6) to KLH (keyhole limpet hemocyanin). 150 to 200 $\mu$g of the antigen protein was administrated to a few tens of intradermal sites of a rabbit 7 to 10 times at two-week intervals, thus immunizing the rabbit (600 to 800 $\mu$g/kg body weight). The first immunization was performed using a mixture of the antigen protein and a complete adjuvant, and the second and subsequent immunizations were performed using a mixture of the antigen protein and an incomplete adjuvant. Next, after the increase in antibody titer against the polypeptide had been confirmed, whole blood was collected from the rabbit, and serum was further collected from the whole blood. The thus-collected serum was used as an antiserum. The concentration of a polyclonal anti-human prorenin receptor antibody (clone name: PRR1) in the antiserum was 0.6 μg/μl. The homology (the alignment score between two different sequences) between the complete amino acid sequence of the human prorenin receptor protein used in the antigen protein and the complete amino acid sequence of a mouse prorenin receptor protein (SEQ ID NO: 10) was 92.29%. Thus, it can be said that the human prorenin receptor antibody contained in the antiserum also binds to the mouse prorenin receptor.

Mouse prorenin receptor protein (SEQ ID NO: 10)

MAVLVVLLFFLVAGALGNEFSILRSPGSVVFRNGNWPIPGDRIPDVAALSM
GFSVKEDLSWPGLAVGNLFHRPRATIMVMVKGVDKLALPAGSVISYPLEN
AVPFSLDSVANSIHSLFSEETPVVLQLAPSEERVYMVGKANSVFEDLSVTL
RQLRNRLFQENSLLNSLPLNSLSRNNEVDLLFLSELQVLHDISSLLSRHK
HLAKDHSPDLYSLELAGLDELGKRYGEDSEQFRDASKILVDALQKFADD
MYSLYGGNAVVELVTVKSFDTSLVRKSRTILEAKQENTQSPYNLAYKYNL
EYSVVFNLVLWIMIGLALAVIITSYNIWNMDPGYDSIIYRMTNQKIRID

(2) Suppression of cancer cell growth in vivo

One × $10^6$ of PK-1 or PANC-1 cells, 30 μl of the antiserum (the antibody: 0.6 μg/μl), and 170 μl of PBS were mixed together. The resultant mixture (the whole amount) was transplanted into an upper right flank region of each of nude mice (n = 10). At this time, the amounts of the cells and the antibody transplanted per kilogram body weight of each mouse were as follows: the cells: 50 × $10^6$; and the antibody: 0.9 mg. Next, after the transplantation, 10 μl of the antiserum (the antibody: 0.6 μg/μl) was administrated into the PK-1- or PANC-1-derived tumor every three days. The amount of the antibody administrated in a single administration was 0.3 mg/kg body weight of the mouse. Then, on Days 15, 18, 21, 24, and 27 after the transplantation, the PK-1- or PANC-1-derived tumor volume in the upper right flank region was measured in the same manner as in Example 7. As a control, the tumor volume was measured in the same manner, except that, instead of the anti-prorenin receptor antibody, a human IgG1 antibody (WAKO) was administrated in an amount of 1 mg/kg body weight of each mouse.

The results thereof are shown in FIGs. 13A and 13B. FIG. 13A is a graph showing the tumor volume in the case where PK-1 was transplanted, and FIG. 13B is a graph showing the tumor volume in the case where the PANC-1 was transplanted. In FIGs. 13A and 13B, the horizontal axis indicates the number of days elapsed after the transplantation, and the vertical axis indicates the tumor volume. In FIGs. 13A and 13B, filled bars indicate the results obtained regarding the anti-prorenin receptor administration group, and open bars indicate the results obtained regarding the human IgG1 antibody administration group. As can be seen from FIGs. 13A and 13B, in both the PK-1-transplanted nude mice and the PANC-1-transplanted nude mice, the tumor volumes on Days 15, 18, 21, 24, and 27 after the transplantation in the anti-prorenin receptor administration group were smaller than those in the human IgG1 antibody administration group. From these results, it was found that cancer growth can be suppressed by using an anti-prorenin receptor antibody in vivo.

[Example 9]

The expression of a prorenin receptor protein in a human pancreatic duct epithelial cell line and in human pancreatic cancer cell lines was examined. The expression of the prorenin receptor protein in culture supernatants of these cell lines was also examined.

As the human pancreatic duct epithelial cell line, HPDE was used. As the human pancreatic cancer cell lines, PK-8, PCI-35, BxPC-3, PK-1, PANC-1, and MIAPaCa-2 were used. Each of the cell lines was cultured in a serum-free medium at 37°C in 5% $CO_2$ for 24 hours. As the serum-free medium, RPMI-1640 (Sigma) was used. After the culture, the culture supernatant was collected. Next, 1 ml of the culture supernatant was concentrated using an Amicon Ultra-0.5 Centrifugal Filter Unit with Ultracel-10 membrane (Millipore), whereby a concentrate containing 20 μg of total protein was obtained. The concentrate was used as a culture supernatant sample.

After the collection of the culture supernatant, the cell line was lysed with a lysis buffer. The cell lysate was centrifuged at 12,500 rpm for 10 minutes, and then, the supernatant was collected. The thus-obtained supernatant was used as a cell lysate sample. Then, the expression of a prorenin receptor protein and a β-actin protein in the culture supernatant sample and the cell lysate sample was examined in the same manner as in the item (1) in Example 3.

The results thereof are shown in FIGs. 14A and 14B. FIG. 14A is a Western Blot photograph showing the expression of the prorenin receptor protein in the cell lysate samples of the respective cell lines. FIG. 14B is a Western Blot photograph showing the expression of the prorenin receptor protein in the culture supernatant samples of the respective cell lines. In FIGs. 14A and 14B, the cell lines used are indicated above the photograph, and the detected proteins are indicated on the left of the photograph. As can be seen from FIG. 14A, the expression of the prorenin receptor protein was observed in the cell lysate samples of all the cell lines. Also, as can be seen from FIG. 14B, the expression of the prorenin receptor protein was observed in the culture supernatant samples of all the cell lines. These results confirm the expression of the prorenin receptor protein in the human pancreatic duct epithelial cell line and the human pancreatic cancer cell lines, and also, in the culture supernatants of these cell lines.

[Example 10]

Whether Wnt signals are suppressed by suppressing the expression of a prorenin receptor was examined.

(1) Expression of phosphorylated LRP6

In the same manner as in Example 2, siRNA (P) RR was transfected into PK-1 (siRNA (P) RR group). After the transfection, a recombinant human Wnt3a was added so that the concentration thereof became 150 ng/ml. Ten minutes after the addition of the recombinant human Wnt3a, PK-1 was collected and lysed with a lysis buffer. Thus, a test sample was prepared. Next, the expression of a prorenin receptor protein and a β-actin protein was examined in the same manner as in the item (1) in Example 3.

The expression level of a phosphorylated LRP6 protein or LRP6 protein in the above test sample was measured in the same manner as in the item (1) in Example 3, except that an anti-phosphorylated LRP6 antibody (Ser 1490, Cell Signaling Technology) or an anti-LRP6 antibody (clone name: C47E12, Cell Signaling Technology) was used instead of the anti-prorenin receptor antibody. Also, the expression levels of the phosphorylated LRP6 protein and the LRP6 protein were measured in the same manner, except that: as a negative control, the siRNA was not transfected and the Wnt3a was not added; as control 1, the siRNA was not transfected; and as control 2, a scrambled siRNA was transfected instead of the siRNA (P) RR.

Furthermore, regarding each sample, the ratio of the expression level of the phosphorylated LRP6 protein to the expression level of the LRP6 protein was determined (this ratio is referred to as the "phosphorylated LRP6 expression level ratio"). Then, assuming that the phosphorylated LRP6 expression level ratio in the negative control was 1, the relative value of the phosphorylated LRP6 expression level ratio in each sample was calculated.

FIG. 15 shows the expression of the prorenin receptor protein and the β-actin protein. FIG. 16 shows the relative values of the phosphorylated LRP6 expression level ratio. FIG. 15 is a Western Blot photograph showing the expression of the prorenin receptor protein and the β-actin protein. In FIG. 15, the siRNA used for the treatment and the presence or absence of the Wnt3a stimulation are indicated below the photograph, and the detected proteins are shown on the left of the photograph. As can be seen from FIG. 15, the expression level of the prorenin receptor protein in the siRNA (P) RR group was lower than those in the negative control and the controls 1 and 2.

FIG. 16 is a graph showing the relative values of the phosphorylated LRP6 expression level ratio. In FIG. 16, the horizontal axis indicates the siRNA used for the treatment and the presence or absence of the Wnt3a stimulation, and the vertical axis indicates the relative value of the phosphorylated LRP6 expression level ratio. As can be seen from FIG. 16, the relative value of the phosphorylated LRP6 expression level ratio after the Wnt3a stimulation in the siRNA (P) RR group was lower than those in the negative control and the controls 1 and 2. From these results, it was confirmed that Wnt signals are suppressed by suppressing the expression of a prorenin receptor protein.

(2) Expression of active β-catenin and Cyclin D1

A test sample was prepared in the same manner as in the above item (1). Next, the expression levels of an active β-catenin protein and a Cyclin D1 protein in the test sample were determined in the same manner as in the item (1) in Example 3, except that an anti-active β-catenin antibody (anti-ABC, Millipore) or an anti-Cyclin D1 antibody was used instead of the anti-prorenin receptor antibody. The expression levels of the active β-catenin protein and the Cyclin D1 protein also were determined in the same manner, except that: as a negative control, a scrambled siRNA was transfected instead of the siRNA (P) RR and the Wnt3a was not added; and as a control, a scrambled siRNA was transfected instead of the siRNA (P) RR. Then, assuming that the expression levels of the active β-catenin protein and the Cyclin D1 protein in the negative control were 1, the relative values of the expression levels of the active β-catenin protein and the Cyclin D1 protein in each sample were determined.

Also, regarding the BxPC-3 and the PANC-1, the relative values of the expression levels of the active β-catenin protein and the Cyclin D1 protein were determined in the same manner.

The results thereof are shown in FIGs. 17A to 17C. FIGs. 17A to 17C are graphs each showing the relative values of the expression levels of the active β-catenin protein and the Cyclin D1 protein. FIG. 17A shows the results obtained when the PK-1 was used. FIG. 17B shows the results obtained when the BxPC-3 was used. FIG. 17C shows the results obtained when the PANC-1 was used. In FIGs. 17A to 17C, the horizontal axis indicates the siRNA used for the treatment and the presence or absence of the Wnt3a stimulation, and the vertical axis indicates the relative expression level. In FIGs. 17A to 17C, filled bars indicate the relative expression levels of the active β-catenin protein, and open bars indicate the relative expression levels of the Cyclin D1 protein.

As can be seen from FIGs. 17A to 17C, the relative expression levels of the active β-catenin protein and the Cyclin D1 protein in the siRNA (P) RR group were significantly lower than those in the control, and were nearly equal to or smaller than those in the negative control. The active β-catenin protein and the Cyclin D1 protein are induced by Wnt signals. Thus, from these results, it was confirmed that Wnt signals are suppressed by suppressing the expression of a prorenin receptor protein.

[Example 11]

PK-1 was seeded into a 6-well plate at a density of $5 \times 10^4$ cells/well. Next, siRNA (P) RR was transfected into the PK-1 in the same manner as in Example 2 (siRNA (P) RR group). After the transfection, a recombinant human Wnt3a was added so that the concentration thereof became 150 ng/ml, and the resultant culture was incubated for 48 hours. After the culture, 100 μl of a water-soluble tetrazolium salt (WST-1) reagent further was added to each well, and the resultant culture was incubated for 2 hours. Then, using a plate reader, the absorbance at 450 nm in each well was measured to determine the proliferation potency. The proliferation potency was determined in the same manner, except that: as a negative control, the siRNA was not transfected and the Wnt3a was not added; as control 1, the siRNA was not transfected; and as control 2, a scrambled siRNA was transfected instead of the siRNA (P) RR.

Then, assuming that the proliferation potency in the negative control was 1, the relative value of the proliferation potency in each sample was determined. Furthermore, regarding each of the BxPC-3 and the PANC-1, the relative value of the proliferation potency was determined in the same manner.

The results thereof are shown in are shown in FIGs. 18A to 18C. FIGs. 18A to 18C are graphs each showing the relative value of the proliferation potency. FIG. 18A shows the results obtained when the PK-1 was used. FIG. 18B shows the results obtained when the BxPC-3 was used. FIG. 18C shows the results obtained when the PANC-1 was used. In FIGs. 18A to 18C, the horizontal axis indicates the siRNA used for the treatment and the presence or absence of the Wnt3a stimulation, and the vertical axis indicates the relative proliferation potency.

As can be seen from FIGs. 18A to 18C, when any of the human pancreatic cancer cell lines was used, the relative proliferation potency in the siRNA (P) RR group was lower than those in the negative control and the controls 1 and 2. From these results, it was confirmed that the proliferation of human pancreatic cancer cell lines is suppressed by suppressing the expression of a prorenin receptor protein.

[Example 12]

Whether apoptosis is induced by suppressing the expression of a prorenin receptor was examined.

(1) Measurement of apoptosis cells

In the same manner as in Example 2, siRNA (P) RR was transfected into PK-1 (siRNA (P) RR group). After the transfection, a recombinant human Wnt3a was added so that the concentration thereof became 150 ng/ml, and the resultant culture was incubated for 48 hours. Next, the cultured PK-1 was centrifuged and collected. The collected PK-1 was suspended in 0.5 ml of ice-cooled 70% ethanol, and the thus-obtained suspension was stored at -20°C for 24 hours. After the storage, the PK-1 was washed with PBS, and then suspended in 10 mg/ml RNase A (Macherey-Nagel)-containing PBS. The thus-obtained suspension was incubated at 37°C for 30 minutes. Thereafter, the suspension was subjected to staining with 1 mg/ml PI (Sigma-Aldrich)-containing PBS at 37°C for 30 minutes.

The stained PK-1 was washed with PBS. Then, the PK-1 was suspended in PBS again. Forward-scattered light (FS), side-scattered light (SS), and the DNA content in this suspension were measured using a DNA flow cytometer (Beckman Coulter, Inc.). Also, on the basis of the DNA content, the proportion of cells in each cell cycle was calculated. As a control, forward-scattered light (FS), side-scattered light (SS), and the DNA content were measured and the proportion of cells in each cell cycle was calculated in the same manner, except that a scrambled siRNA was transfected instead of the siRNA (P) RR. Furthermore, regarding PANC-1, the proportion of cells in each cell cycle was calculated in the same manner.

FIG. 19 shows dot plots each showing the FS and the SS. FIG. 20 is a histogram showing the DNA content. FIGs. 21A and 21B are graphs each showing the proportion of cells in each cell cycle. The dot plots shown in FIG. 19 show the proportion of apoptosis cells. In FIG. 19, the horizontal axis indicates the SS, and the vertical axis indicates the FS, and the number shown inside each dot plot indicates the proportion of apoptosis cells. As can be seen from FIG. 19, in the siRNA (P) RR group, the proportion of apoptosis cells, which are cells included in the upper right quarter of the dot plot, was greater than that in the control.

FIG. 20 is a histogram showing the DNA content. In FIG. 20, the horizontal axis indicates the fluorescence intensity of the PI, and the vertical axis indicates the count. Also, in FIG. 20, the filled histogram indicates the result obtained regarding the siRNA (P) RR group, and the open histogram indicates the result obtained regarding the control. As can be seen from FIG. 20, in the siRNA (P) RR group, apoptosis cells having a relatively low DNA content as indicated with the arrow were observed. In contrast, in the control, apoptosis cells were not observed.

FIGs. 21A and 21B are graphs each showing the proportion of cells in each cell cycle. FIG. 21A shows the results obtained when the PK-1 was used. FIG. 21B shows the results obtained when the PANC-1 was used. In FIGs. 21A and 21B, the horizontal axis indicates the siRNA used for the treatment, and the vertical axis indicates the proportion of cells in each cell cycle.

As can be seen from FIGs. 21A and 21B, when any of the human pancreatic cancer cell lines was used, the proportion of SubG1 group cells, which are apoptosis cells, in the siRNA (P) RR group was greater than that in the control. From these results, it was confirmed that apoptosis of human pancreatic cancer cell lines is induced by suppressing the expression of a prorenin receptor protein.

(2) Measurement of activation of caspase 3

PK-1 was cultured and collected in the same manner as in the above item (1). The activity of caspase 3 was measured using an APOPCYTE (Kit name: Caspase-3 Colorimetric Assay Kit, Medical & Biological laboratories) in accordance with its protocol. As a control, the activity of caspase 3 was measured in the same manner, except that a scrambled siRNA was transfected instead of the siRNA (P) RR. Also, regarding the BxPC-3 and the PANC-1, the activity of the caspase 3 was measured in the same manner.

The results thereof are shown in FIGs. 22A to 22C. FIGs. 22A to 22C are graphs each showing the activity of the caspase 3. FIG. 22A shows the results obtained when the PK-1 was used. FIG. 22B shows the results obtained when the BxPC-3 was used. FIG. 22C shows the result obtained when the PANC-1 was used. In FIGs. 22A to 22C, the horizontal axis indicates the siRNA used for the treatment, and the vertical axis indicates the activity of the caspase 3. As can be seen from FIGs. 22A to 22C, when any of the human pancreatic cancer cell lines was used, the activity of the caspase 3 inducing apoptosis in the siRNA (P) RR group was higher than that in the control. From these results, it was confirmed that caspase 3, which induces apoptosis, is activated by suppressing the expression of a prorenin receptor protein.

[Example 13]

The increase in the concentration of the prorenin receptor in the plasma of brain tumor patients was examined.

Plasma was collected from blood of each of the following subjects: healthy males (n = 4), healthy females (n = 2), male brain tumor patients (n = 5), and female brain tumor patients (n = 10). The concentration of the prorenin receptor in the plasma of each subject was measured in the same manner as in Example 1.

The results thereof are shown in FIG. 23. FIG. 23 is a graph showing the concentration of the prorenin receptor in the plasma. In FIG. 23, the horizontal axis indicates the type of the test sample, and the vertical axis indicates the concentration of the prorenin receptor. As can be seen from FIG. 23, in both the male and female subjects, the concentrations of the prorenin receptor in the plasma of the brain tumor patients (Brain tumor) were significantly higher than those in the plasma of the healthy subjects (Normal). From these results, it was found that there is a correlation between the occurrence of the brain tumor and the increase in the concentration of the prorenin receptor in the plasma regardless of the sex of a subject, which demonstrates that the prorenin receptor in plasma serves as a brain tumor marker.

While the invention has been described with reference to the embodiments and the examples, the invention is not limited to these embodiments and examples. Various changes which are understood by those skilled in the art in the scope of the present invention can be applied to the configuration and detail of the present invention.

As described above, the present disclosure allows a morbidity risk of a cancer to be tested by measuring a prorenin receptor expression level. Moreover, a prorenin receptor can be a target of a cancer. Thus, the screening method according to the present disclosure allows a candidate substance for use in a cancer therapy to be obtained using a prorenin receptor as a target. Therefore, the cancer marker according to the present disclosure is really useful marker in a clinical field and a biochemical field.

[Sequence Listing]

T13031WO_2013.09.06_ST25.txt

## Claims

1. An antibody to a prorenin receptor or an antigen binding fragment of the antibody, wherein the antibody or the antigen binding fragment binds a region extending from the $200^{th}$ to $213^{rd}$ amino acid residues in an amino acid sequence (SEQ ID NO: 6) of a human prorenin receptor protein.

2. A drug for use in a method of treating cancer, comprising the antibody or the antigen binding fragment according to claim 1.

3. The drug for use according to claim 2, wherein the cancer is a gastrointestinal cancer or a brain tumor.

4. The drug for use according to claim 3, wherein the gastrointestinal cancer is at least one selected from the group consisting of a pancreatic cancer, a gastric cancer, a large bowel cancer, and a liver cancer.

5. The drug for use according to claims 3, wherein the cancer is a pancreatic cancer

6. The drug for use according to claims 3, wherein the brain tumor is at least one selected from the group consisting of glioma, astrocytoma, primary central nervous system malignant lymphoma, and cavernous hemangioma.

## Patentansprüche

1. Antikörper gegen einen Proreninrezeptor oder ein Antigen-Bindungsfragment des Antikörpers, wobei der Antikörper oder das Antigen-Bindungsfragment einen Bereich bindet, der sich von den 200. bis 213. Aminosäureresten in einer Aminosäuresequenz (SEQ ID NO: 6) eines menschlichen Proreninrezeptorproteins erstreckt.

2. Arzneimittel zur Verwendung in einem Verfahren zur Behandlung von Krebs, umfassend den Antikörper oder das Antigen-Bindungsfragment nach Anspruch 1.

3. Arzneimittel zur Verwendung nach Anspruch 2, wobei der Krebs ein Magen-Darm-Krebs oder ein Hirntumor ist.

4. Arzneimittel zur Verwendung nach Anspruch 3, wobei der Magen-Darm-Krebs mindestens einer ausgewählt aus der Gruppe bestehend aus einem Bauchspeicheldrüsenkrebs, einem Magenkrebs, einem Dickdarmkrebs und einem Leberkrebs ist.

5. Arzneimittel zur Verwendung nach Anspruch 3, wobei der Krebs ein Bauchspeicheldrüsenkrebs ist.

6. Arzneimittel zur Verwendung nach Anspruch 3, wobei der Hirntumor mindestens einer ausgewählt aus der Gruppe bestehend aus Gliom, Astrozytom, malignem Lymphom des primären Zentralnervensystems und kavernösem Hämangiom ist.

## Revendications

1. Anticorps dirigé contre un récepteur de la prorénine ou un fragment de liaison à l'antigène de l'anticorps, dans lequel l'anticorps ou le fragment de liaison à l'antigène se lie à une région s'étendant du 200ème au 213ème résidus d'acide aminé dans une séquence d'acides aminés (SEQ ID NO: 6) d'une protéine réceptrice de prorénine humaine.

2. Médicament pour utilisation dans une méthode de traitement du cancer, comprenant l'anticorps ou le fragment de liaison à l'antigène selon la revendication 1.

3. Médicament pour utilisation selon la revendication 2, dans lequel le cancer est un cancer gastro-intestinal ou une tumeur cérébrale.

4. Médicament pour utilisation selon la revendication 3, dans lequel le cancer gastro-intestinal est au moins un groupe choisi dans le groupe constitué d'un cancer du pancréas, d'un cancer gastrique, d'un cancer du gros intestin et d'un cancer du foie.

5. Médicament pour utilisation selon la revendication 3, dans lequel le cancer est un cancer du pancréas.

6. Médicament pour utilisation selon la revendication 3, dans lequel la tumeur cérébrale est au moins une sélectionnée dans le groupe comprenant le gliome, l'astrocytome, le lymphome malin du système nerveux central primaire et l'hémangiome caverneux.

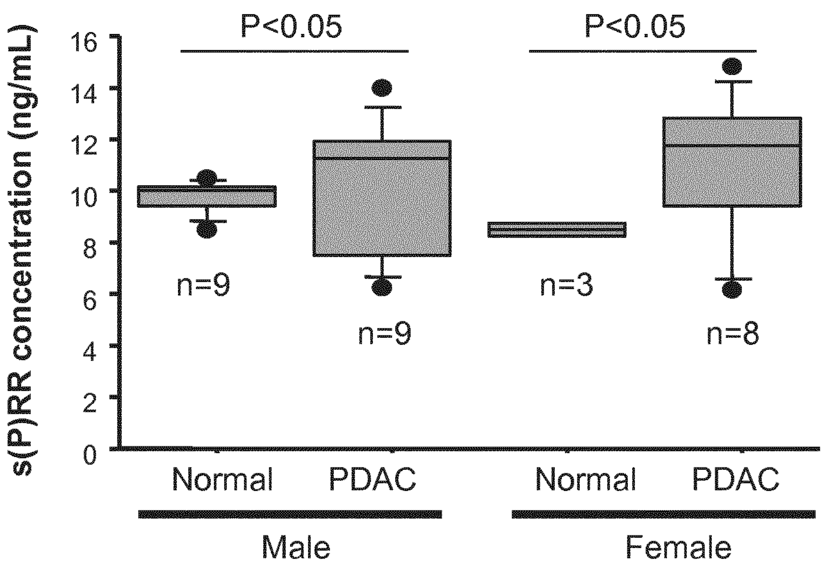
s(P)RR concentration (ng/mL)
0
2
4
6
8
10
12
14
16
P<0.05
P<0.05
n=9
n=9
n=3
n=8
Normal
PDAC
Normal
PDAC
Male
Female

FIG. 1A

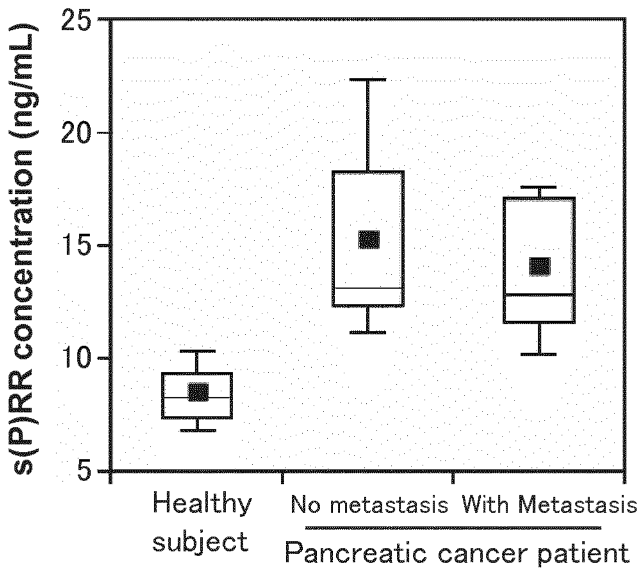
s(P)RR concentration (ng/mL)
5
10
15
20
25
Healthy subject
No metastasis
With Metastasis
Pancreatic cancer patient

FIG. 1B

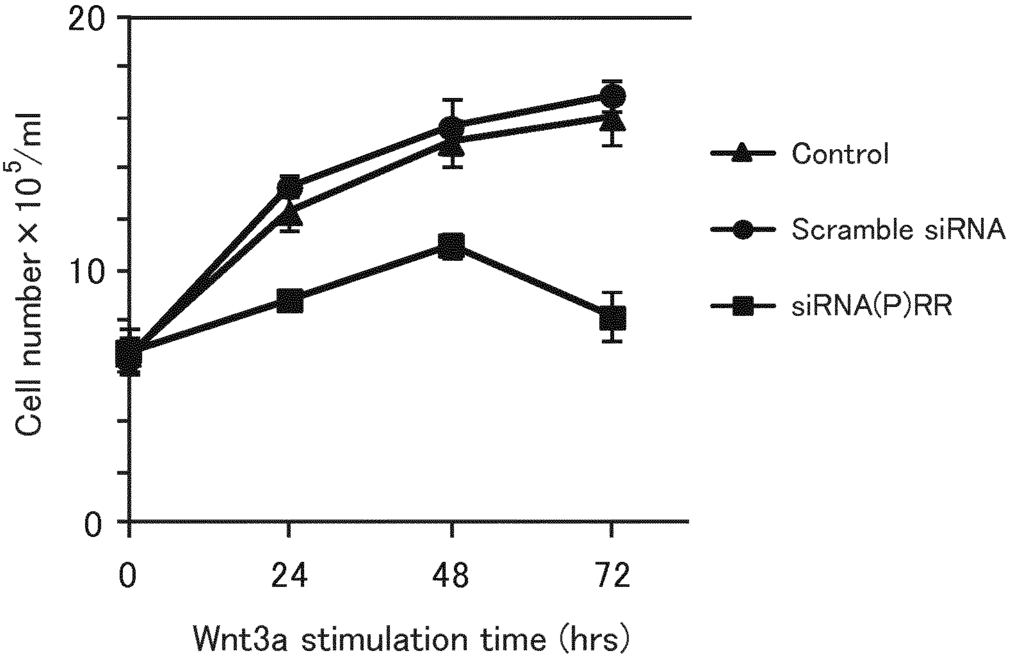
20
10
0
Cell number × $10^5$/ml
0
24
48
72
Wnt3a stimulation time (hrs)
Control
Scramble siRNA
siRNA(P)RR

FIG. 2

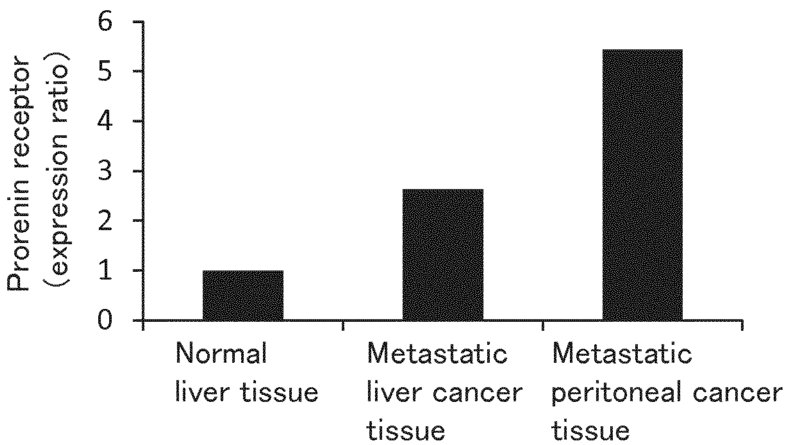
6
5
4
3
2
1
0
Prorenin receptor (expression ratio)
Normal liver tissue
Metastatic liver cancer tissue
Metastatic peritoneal cancer tissue

FIG. 3

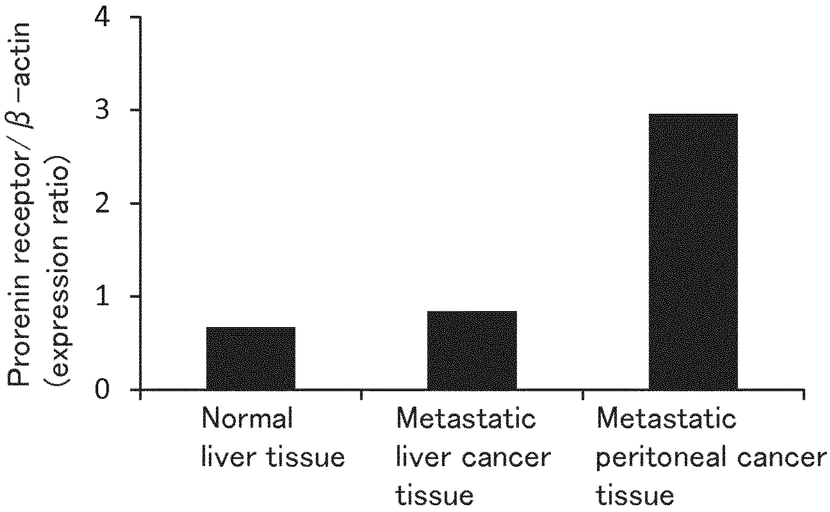
Prorenin receptor/β-actin
(expression ratio)
0
1
2
3
4
Normal liver tissue
Metastatic liver cancer tissue
Metastatic peritoneal cancer tissue

FIG. 4

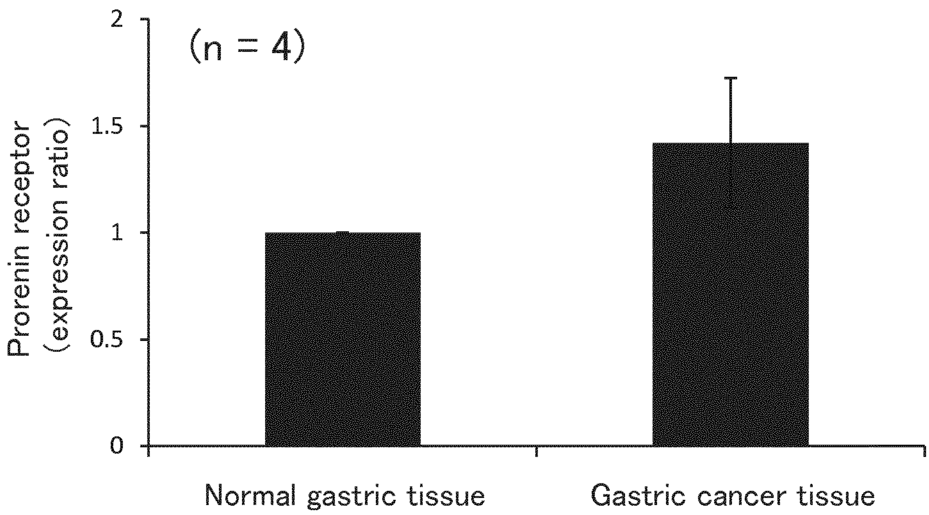
Prorenin receptor
(expression ratio)
(n = 4)
0
0.5
1
1.5
2
Normal gastric tissue
Gastric cancer tissue

FIG. 5

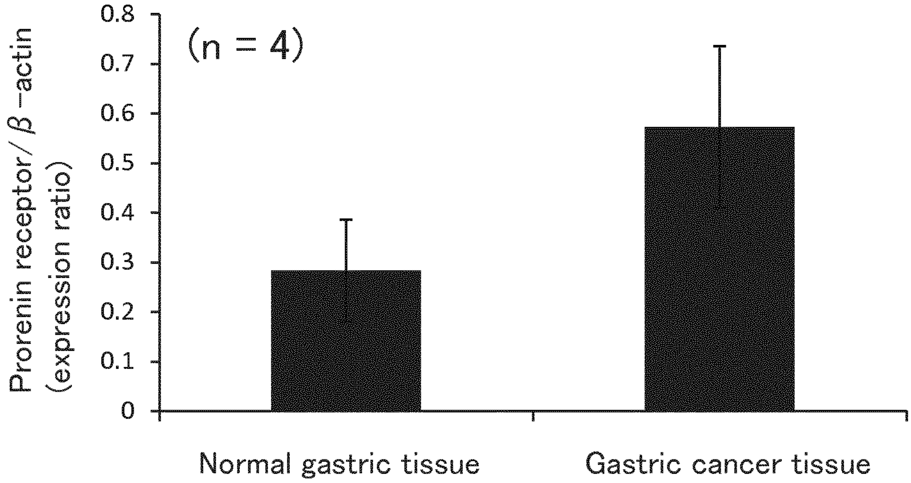
Prorenin receptor/β-actin
(expression ratio)
0.8
0.7
0.6
0.5
0.4
0.3
0.2
0.1
0
(n = 4)
Normal gastric tissue
Gastric cancer tissue

FIG. 6

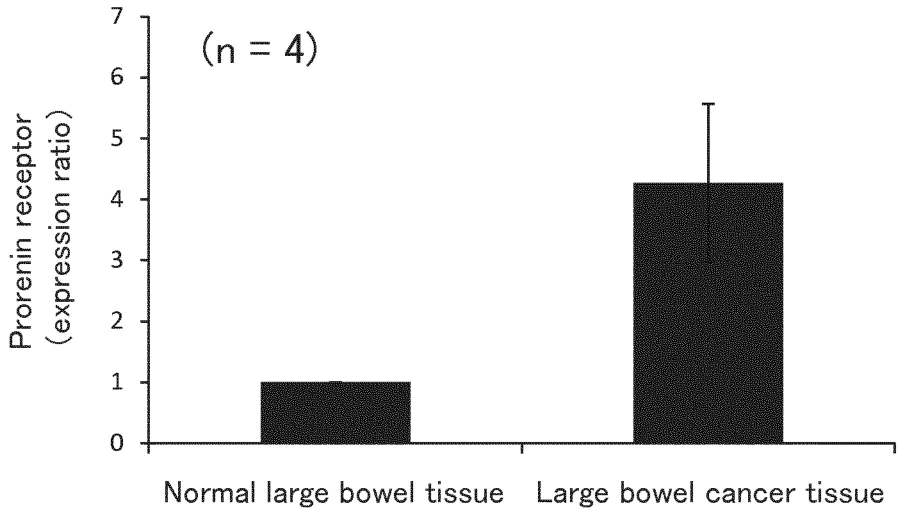
Prorenin receptor
(expression ratio)
7
6
5
4
3
2
1
0
(n = 4)
Normal large bowel tissue
Large bowel cancer tissue

FIG. 7

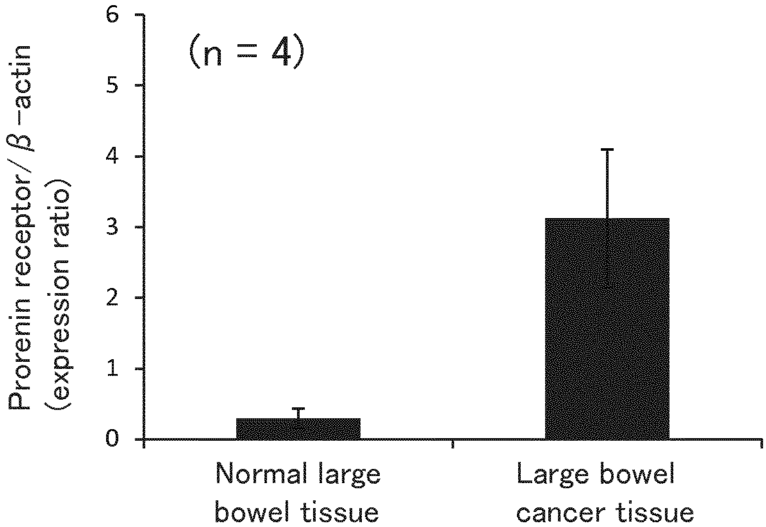

Prorenin receptor/β-actin
(expression ratio)
6
5
4
3
2
1
0
(n = 4)
Normal large bowel tissue
Large bowel cancer tissue


FIG. 8

Pancreatic cancer tissue

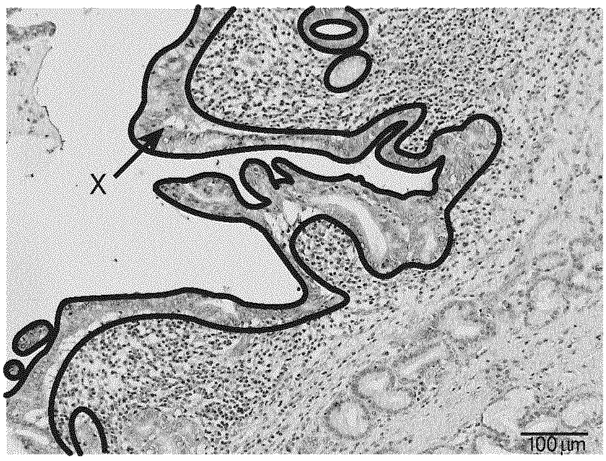


FIG. 9A

Pancreatic duct epithelial tissue (normal)

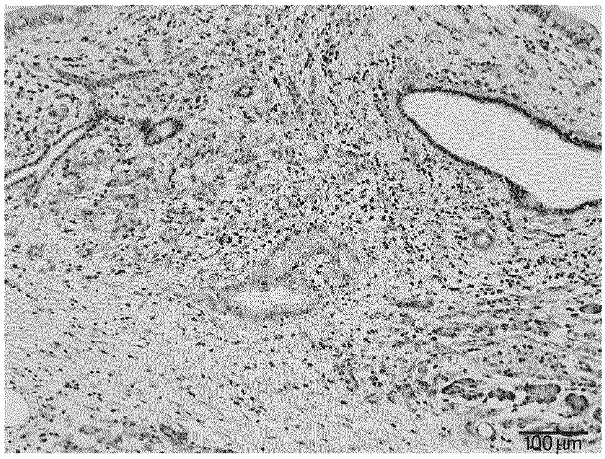


FIG. 9B

Preneoplastic lesion

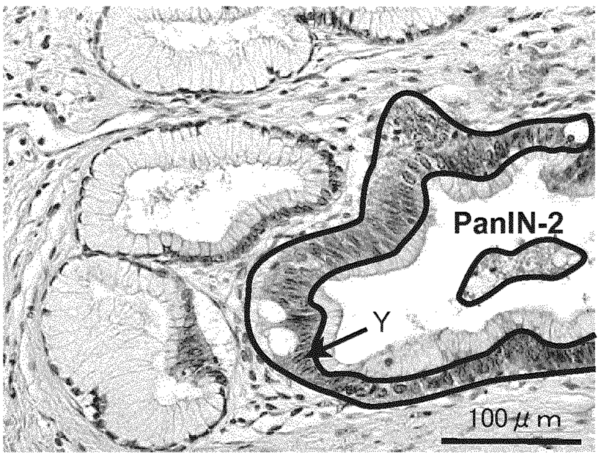


FIG. 9C

Preneoplastic lesion

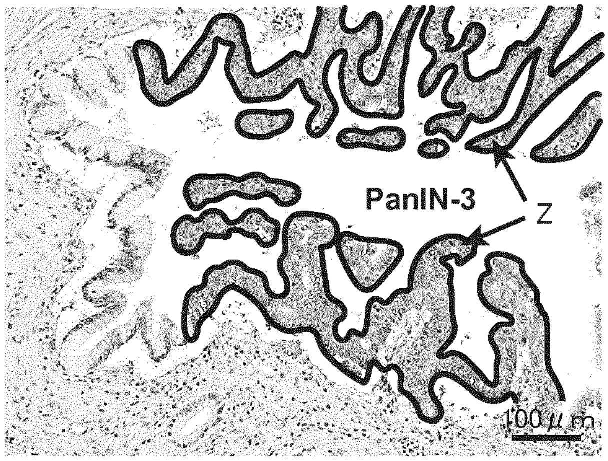


FIG. 9D

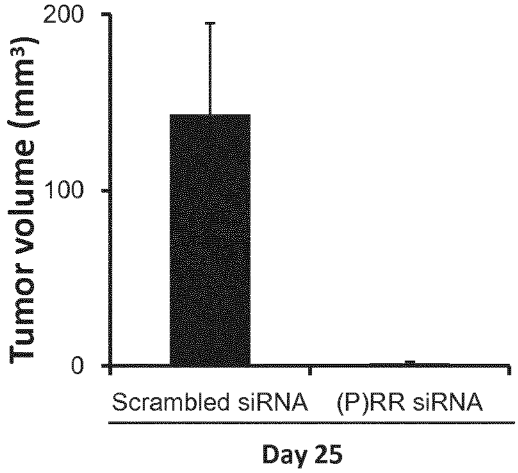


FIG. 10A

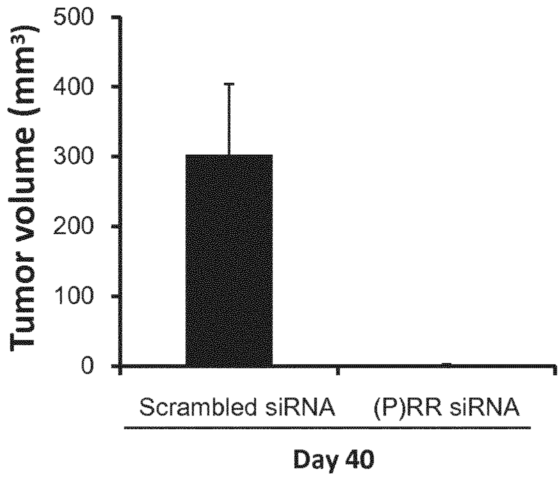


FIG. 10B

Scrambled siRNA

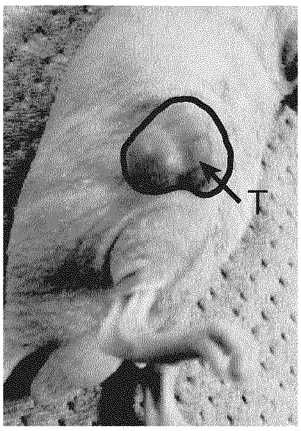


FIG. 11A

(P)RR siRNA

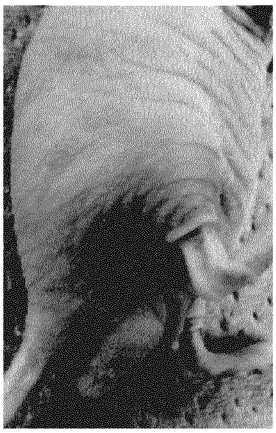

FIG. 11B

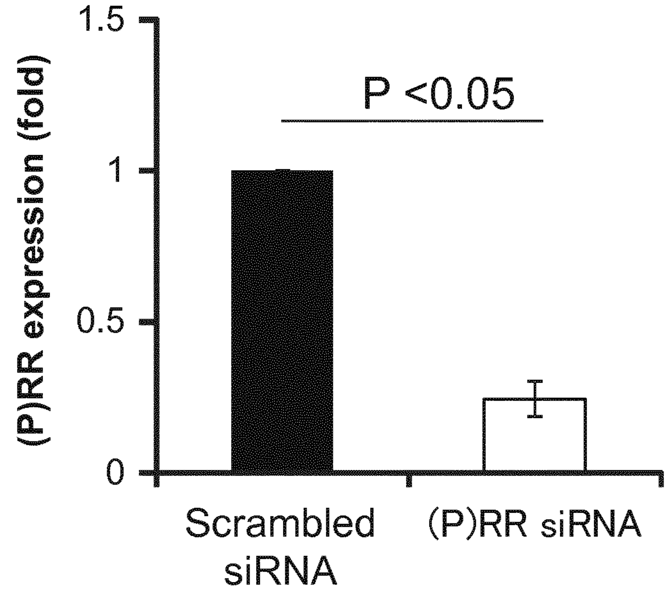


FIG. 12

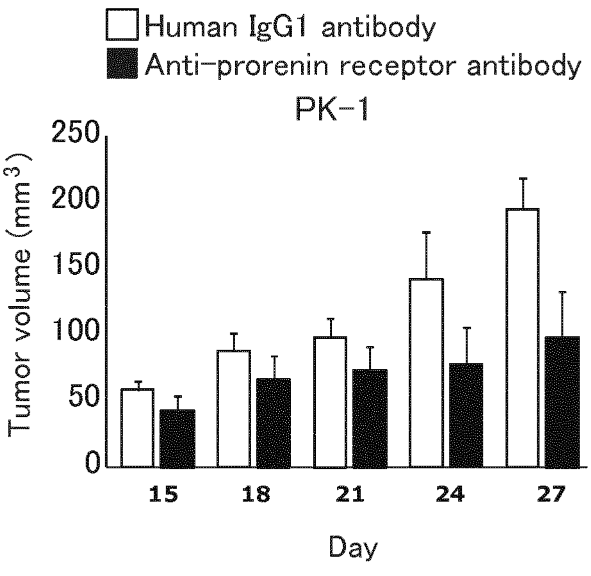


FIG. 13A

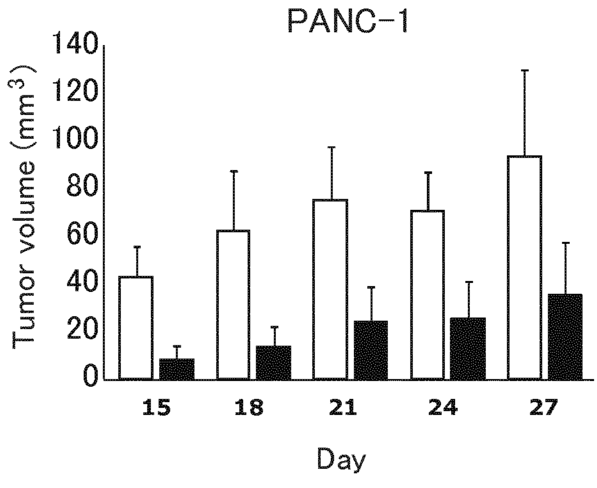


FIG. 13B

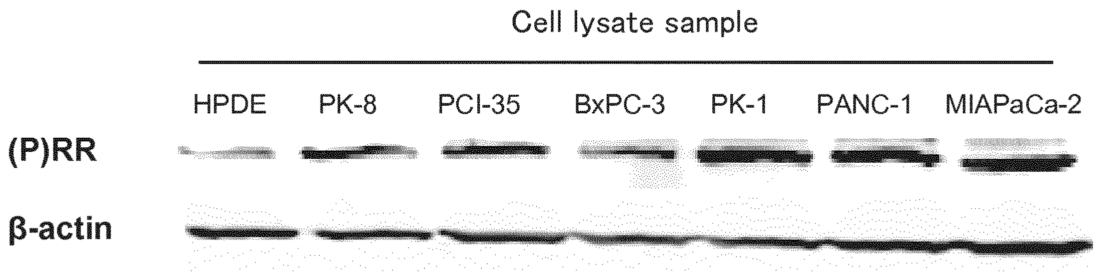


FIG. 14A

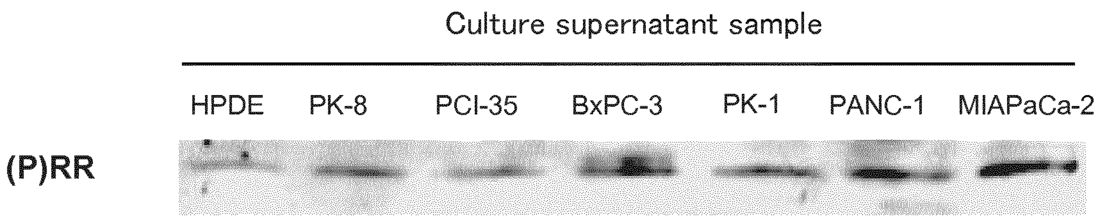


FIG. 14B

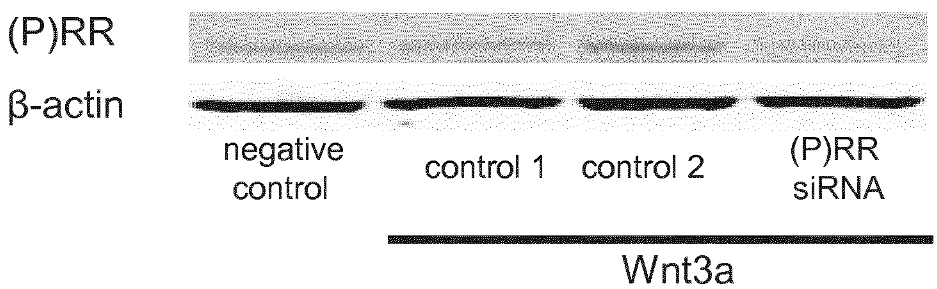


FIG. 15

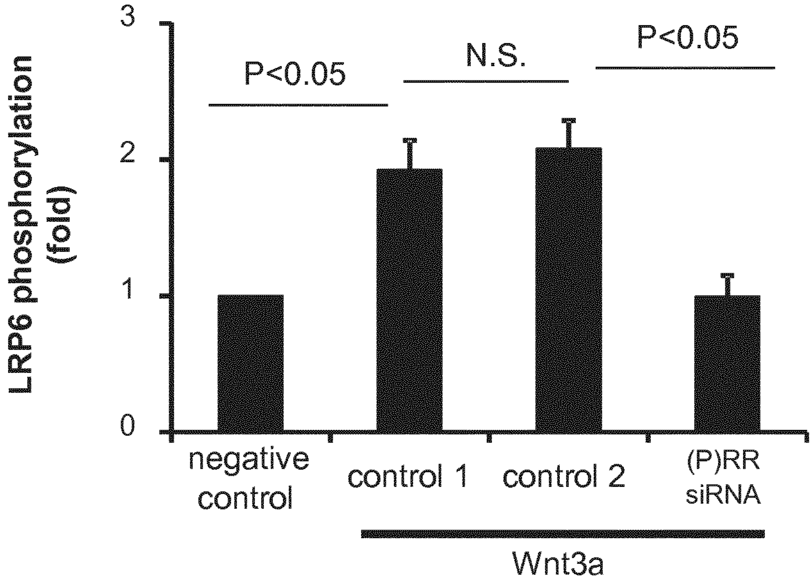
LRP6 phosphorylation
(fold)
3
2
1
0
P<0.05
N.S.
P<0.05
negative
control
control 1
control 2
(P)RR
siRNA
Wnt3a

FIG. 16

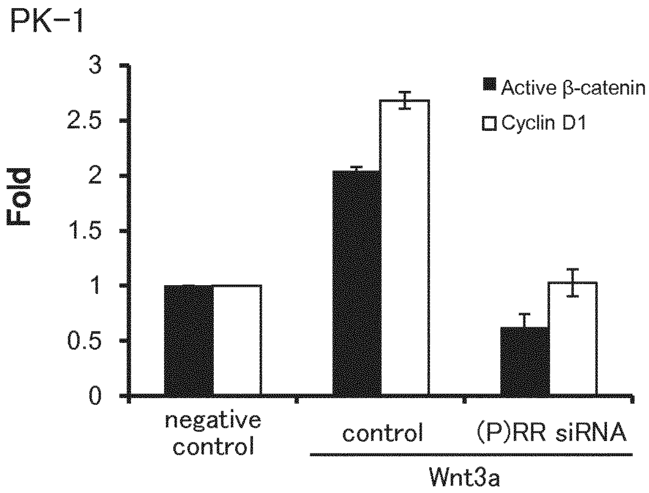


FIG. 17A

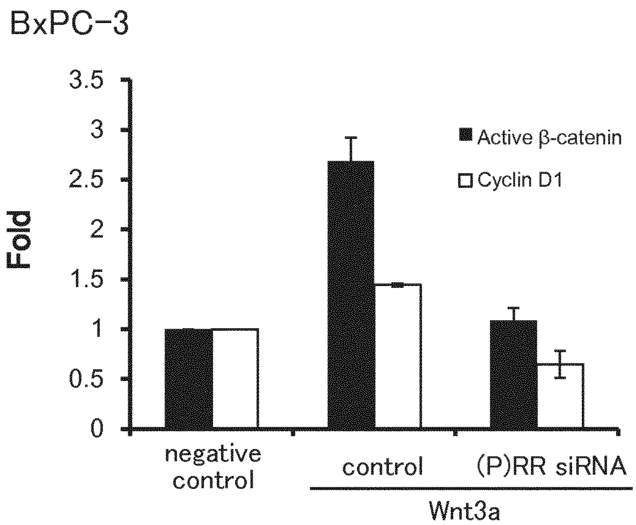


FIG. 17B

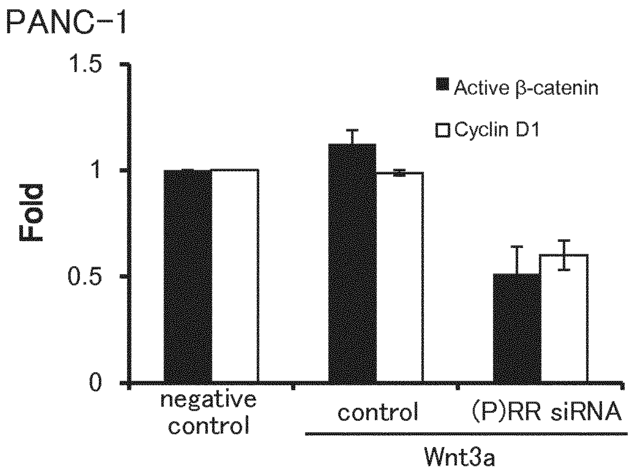


FIG. 17C

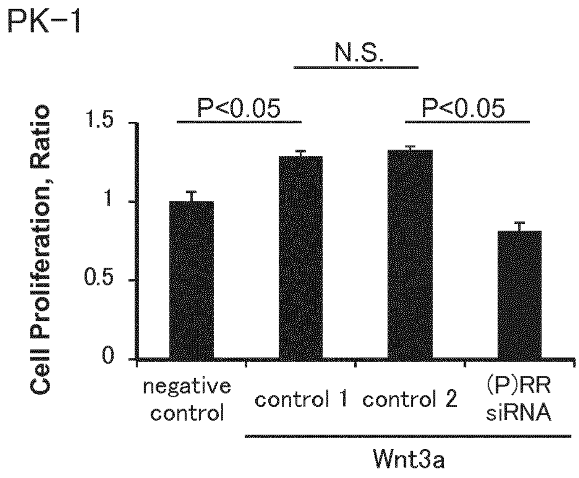


FIG. 18A

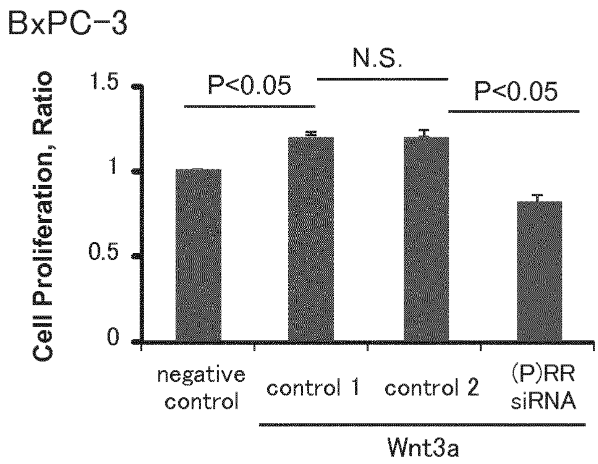


FIG. 18B

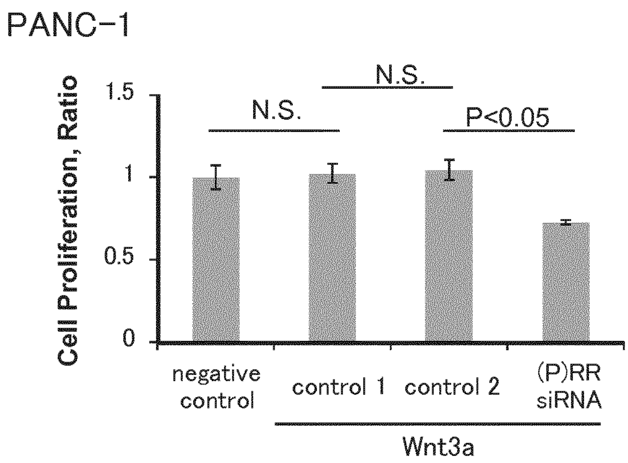


FIG. 18C

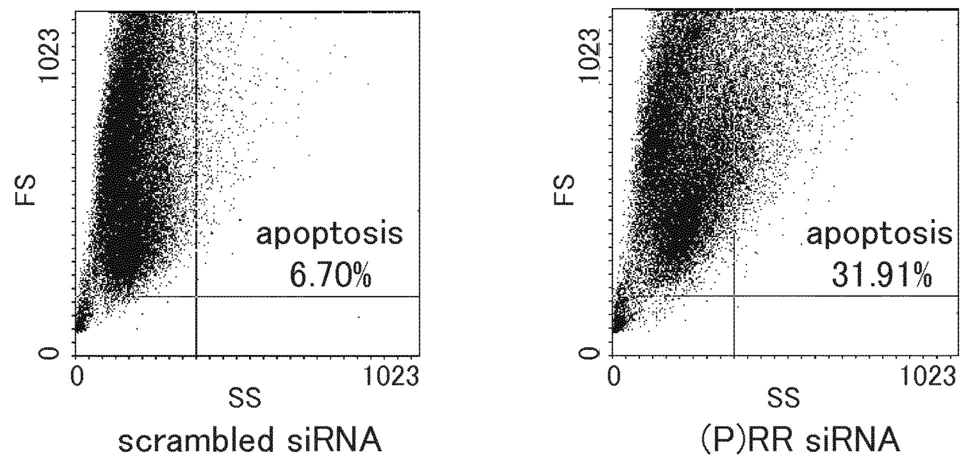
1023
FS
0
apoptosis
6.70%
0
1023
SS
scrambled siRNA
1023
FS
0
apoptosis
31.91%
0
1023
SS
(P)RR siRNA

FIG. 19

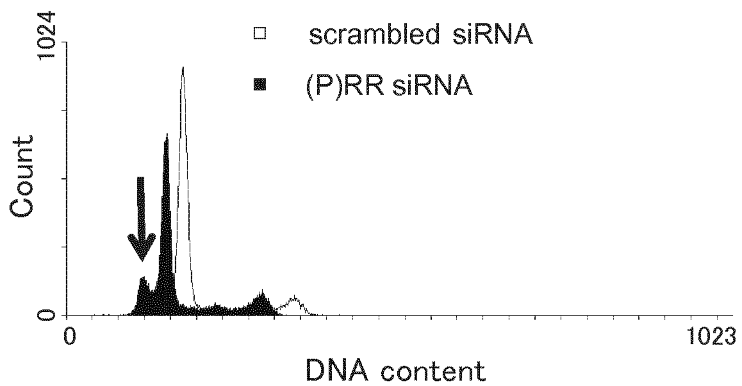
1024
Count
0
scrambled siRNA
(P)RR siRNA
0
1023
DNA content

FIG. 20

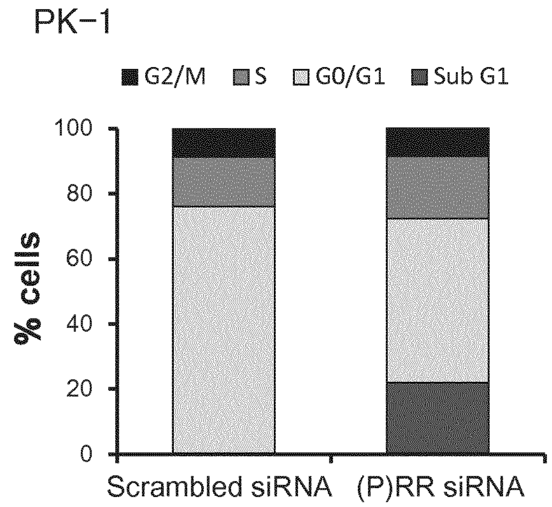


FIG. 21A

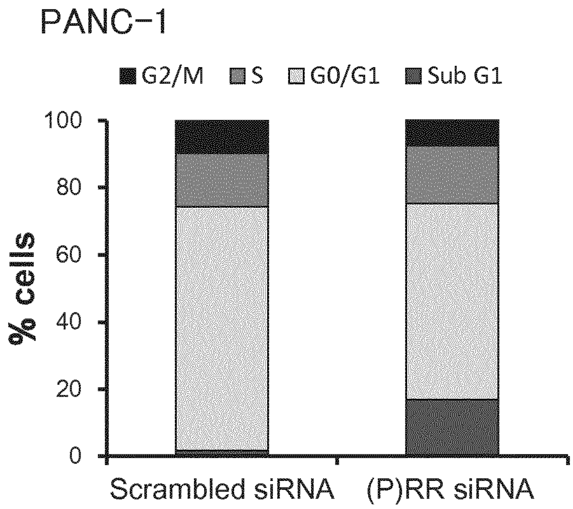


FIG. 21B

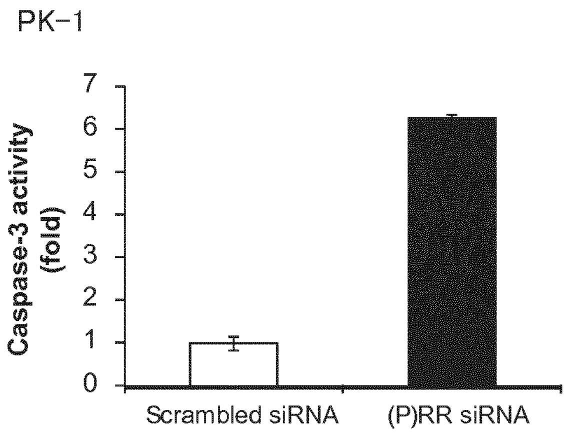


FIG. 22A

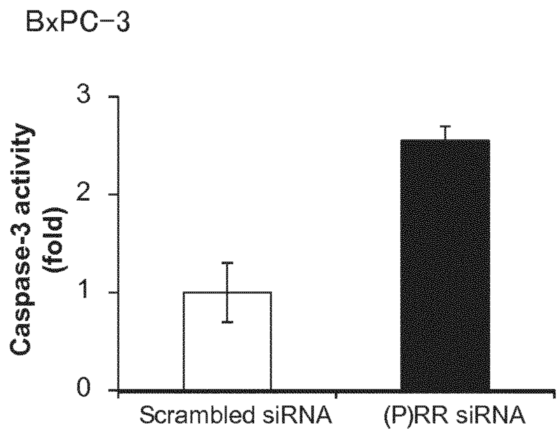


FIG. 22B

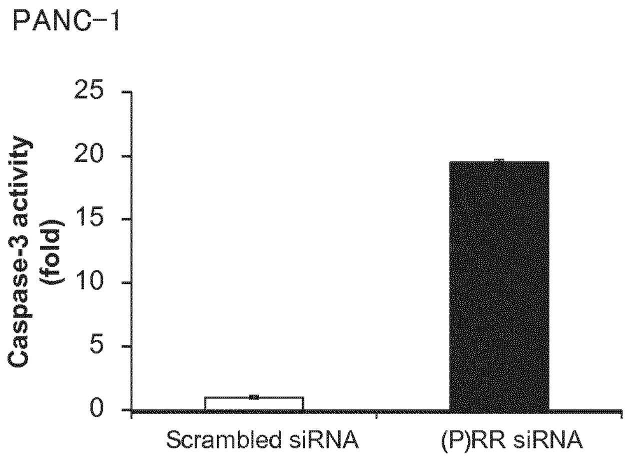


FIG. 22C

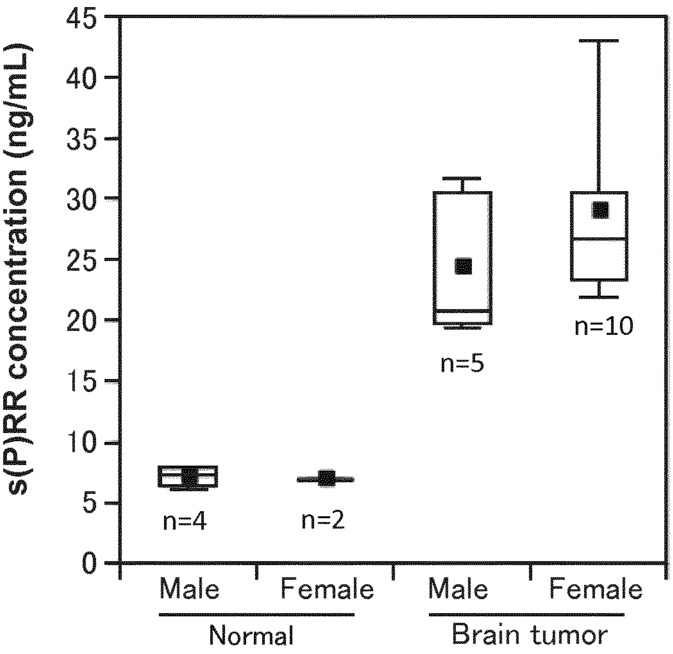
s(P)RR concentration (ng/mL)
45
40
35
30
25
20
15
10
5
0
n=4
n=2
n=5
n=10
Male
Female
Male
Female
Normal
Brain tumor

FIG. 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012045587 A
- US 2011263441 A
- WO 9934823 A
- WO 2013124406 A
- JP 2012199508 A


### Non-patent literature cited in the description


- **DUFFY MJ et al.** Tumor markers in pancreatic cancer: a European Group on Tumor Markers (EGTM) status report. *Annals of Oncology, Oxford Journal,* March 2010, vol. 21 (3), 441-447
- Feature, Diagnosis and treatment of pancreatic cancer, Is it possible to early diagnose pancreatic cancer? Tumor marker. **WATANABE HIROYUKI et al.** Geka chiryo (surgical therapy). Nagai Shoten Co., Ltd, September 2007, vol. 97, 250-257
- **JUILLERAT-JEANNERET, L et al.** Renin and angiotensinogen expression and functions in growth and apoptosis of human glioblastoma. *British journal of cancer,* 2004, vol. 90 (5), 1059-68